# EUROPEAN PATENT SPECIFICATION

(11) **EP 1 617 820 B1**
(45) Date of publication and mention of the grant of the patent: **21.03.2018**
(21) Application number: 04727320.6
(22) Date of filing: 14.04.2004
(51) Int. Cl.: A61K 9/16, A61K 47/18, A61K 9/14

(54) **DRY POWER INHALER DEVICES AND DRY POWER FORMULATIONS FOR ENHANCING DOSING EFFICIENCY**
TROCKENPULVERINHALATOREN UND TROCKENPULVERFORMULIERUNGEN ZUM ERHÖHEN DER DOSIERLEISTUNG
INHALATEURS A POUDRE SECHE ET FORMULATIONS DE POUDRES SECHES DESTINEES A AUGMENTER L'EFFICACITE DE DOSAGE

(30) Priority: 14.04.2003 US 413022; 17.07.2003 US 621964; 15.09.2003 GB 0321612
(43) Date of publication of application: 25.01.2006
(73) Proprietor: Vectura Limited, Chippenham, Wiltshire SN14 6FH (GB)
(72) Inventor: STANIFORTH, John, Chippenham Wiltshire SN14 6FH (GB); MORTON, David, Chippenham Wiltshire SN14 6FH (GB); TOBYN, Michael, Chippenham Wiltshire SN14 6FH (GB); EASON, Stephen, Chippenham Wiltshire SN14 6FH (GB); HARMER, Quentin, Chippenham Wiltshire SN14 6FH (GB); GANDERTON, David, Chippenham Wiltshire SN14 6FH (GB)
(74) Representative: CMS Cameron McKenna Nabarro Olswang LLP
(86) International application number: PCT/GB2004/001628
(87) International publication number: WO 2004/093848

(56) References cited:
- EP-A- 1 213 012
- WO-A-01/13893
- WO-A-01/78693
- WO-A-91/16038
- WO-A-02/067902
- US-A- 6 045 828
- US-A1- 2002 071 871
- US-A1- 2003 055 034

## Description

The present invention is as claimed in claims 1-14, and relates to enhancing the dosing efficiency of pharmaceutical dry powder formulations administered by pulmonary inhalation. In particular, the present invention relates to the provision of dry powder inhalers and dry powder compositions which reproducibly achieve a much higher delivered dose of the pharmaceutically active agent than currently achieved.

US6,045,828 discloses micronised dry powder formulations suitable for pulmonary administration, wherein such powders are obtained by for example, jet-milling a lyphilisate of an active agent and one or more additives, such as phospholipids. There is however no mention of metal stearates as additives.

WO91/16038 discloses pharmaceutical aerosol formulations of a biologically active polypeptide which is a human interferon or a human interleukin. Solid micronised polypeptide particles are obtained by for example jet-milling in the presence of one or more additives such as sorbitol and albumin. Metal stearates are not disclosed as additives.

WO01/78693 discloses a formulation, preferably in the form of "hard pellets", for use in a dry powder inhaler for efficacious delivery of active ingredients into the low respiratory tract of patients suffering from pulmonary diseases such as asthma.

Detailed studies of powder behaviour and performance has enabled the inventors to ascertain how to balance the various factors that affect dosing efficiency, allowing them to achieve consistent, reproducible and high delivered dose values.

The metered dose (MD) of a dry powder formulation is the total mass of active agent present in the metered form presented by the inhaler device in question. For example, the MD might be the mass of active agent present in a capsule for a Cyclohaler (trade mark), or in a foil blister in an Aspirair (trade mark) device.

The emitted dose (ED) is the total mass of the active agent emitted from the device following actuation. It does not include the material left inside or on the surfaces of the device. The ED is measured by collecting the total emitted mass from the device in an apparatus frequently identified as a dose uniformity sampling apparatus (DUSA), and recovering this by a validated quantitative wet chemical assay.

The fine particle dose (FPD) is the total mass of active agent which is emitted from the device following actuation which is present in an aerodynamic particle size smaller than a defined limit. This limit is generally taken to be 5µm if not expressly stated to be an alternative limit, such as 3µm or 1µm, etc. The FPD is measured using an impactor or impinger, such as a twin stage impinger (TSI), multi-stage impinger (MSI), Andersen Cascade Impactor or a Next Generation Impactor (NGI). Each impactor or impinger has a pre-determined aerodynamic particle size collection cut points for each stage. The FPD value is obtained by interpretation of the stage-by-stage active agent recovery quantified by a validated quantitative wet chemical assay where either a simple stage cut is used to determine FPD or a more complex mathematical interpolation of the stage-by-stage deposition is used.

The fine particle fraction (FPF) is normally defined as the FPD divided by the ED and expressed as a percentage. Herein, the FPF of ED is referred to as FPF(ED) and is calculated as FPF(ED) = (FPD/ED) x 100%.

The fine particle fraction (FPF) may also be defined as the FPD divided by the MD and expressed as a percentage. Herein, the FPF of MD is referred to as FPF(MD), and is calculated as FPF(MD) = (FPD/MD) x 100%.

The FPF(MD) can also be termed the 'Dose Efficiency' and is the amount of the dose of the pharmaceutical dry powder formulation which, upon being dispensed from the delivery device, is below a specified aerodynamic particle size.

It is well known that particle impaction in the upper airways of a subject is predicted by the so-called impaction parameter. The impaction parameter is defined as the velocity of the particle times the square of its aerodynamic diameter. Consequently, the probability associated with delivery of a particle through the upper airways region to the target site of action, is related to the square of its aerodynamic diameter. Therefore, delivery to the lower airways, or the deep lung is dependant on the square of its aerodynamic diameter, and smaller aerosol particles are very much more likely to reach the target site of administration in the user and therefore able to have the desired therapeutic effect.

Particles having aerodynamic diameters in the range of 5µm to 2µm will generally be deposited in the respiratory bronchioles whereas smaller particles having aerodynamic diameters in the range of 3 to 0.05µm are likely to be deposited in the alveoli. So, for example, high dose efficiency for particles targeted at the alveoli is predicted by the dose of particles below 3µm, with the smaller particles being most likely to reach that target site.

At present, many of the commercially available dry powder inhalers exhibit very poor dosing efficiency, with sometimes as little as 10% of the active agent present in the dose actually being properly delivered to the user so that it can have a therapeutic effect. Whilst isolated incidences of high percentages of dose delivered have been possible in the prior art, it has not previously been possible to repeatedly and consistently achieve a dose efficiency at 5 or 3µm of 70% or more.

The reason for this lack of dosing efficiency is that a proportion of the active agent in the dose of dry powder tends to be effectively lost at every stage the powder goes through from expulsion from the delivery device to deposition in the lung. For example, substantial amounts of material may remain in the device. Material may be lost in the throat of the subject due to excessive plume velocity. However, it is frequently the case that a high percentage of the dose delivered exists in particulate forms of aerodynamic diameter in excess of that required.

Therefore, the present invention provides ways in which the loss of the pharmaceutically active agent is reduced at each of these stages, so that a high dosing efficiency can be achieved.

In the past, efforts to increase dosing efficiency and to obtain greater dosing reproducibility have tended to focus on preventing the formation of agglomerates of fine active particles. Such agglomerates increase the effective size of these particles and therefore prevent them from reaching the lower respiratory tract or deep lung, where the active particles should be deposited in order to have their desired therapeutic effect.

However, it has now been recognised that other factors affect the loss of active agent during known delivery of powder formulations.

Firstly, it is common for at least some of the dose of powder formulation, including some of the active agent, to be left in the dispensing device or in the dose storage container, such as a blister or capsule after use. There are several points at which such retention in the device may occur and these will be discussed in greater detail below.

Secondly, the dynamics of the cloud of powder released by the dispensing device will affect the amount of the powder and therefore of the active agent which will become deposited in the throat of the user. Once again, active agent is effectively lost if it is deposited in the throat as it will not have any therapeutic effect. It has been found that the shape of the plume of powder formed by the device, and the velocity of the active particles in particular, will affect the deposition in the throat. This will be discussed in greater detail below.

Thirdly, as recognised in the art, the fine particles of active agent tend to agglomerate and if these agglomerates are not broken up upon actuation of the dispensing device, the active agent particles will not reach the desired part of the lung. It has been found that the deagglomeration of the fine powder particles can be greatly enhanced by the addition of force control agents which reduce particle cohesion to allow agglomerates to break up more easily, as well as by the methods used to prepare the particles.

All of the ways of improving dosing efficiency disclosed herein may be added to techniques already known and used in the art in order to achieve a dosing efficiency at 5µm of preferably at least 70%, preferably at least 75%, more preferably at least 80%, more preferably at least 85%, more preferably at least 90%, and most preferably at least 95%. The improvements may also lead to a dosing efficiency at 3µm of preferably at least 60%, preferably at least 70%, more preferably at least 75%, more preferably at least 80%, more preferably at least 85%, and most preferably at least 90%. The improvements may also allow one to achieve a dosing efficiency at 2µm of preferably at least 40%, preferably at least 50%, more preferably at least 55%, more preferably at least 60%, and most preferably at least 70%. These efficiencies are far greater than anything consistently achieved prior to this invention using simple, practical and cost effective methods of preparation, which would be suitable for the pharmaceutical industry and the methods used are described in more detail below. These methods are in stark contrast to the known technologies for producing high performance, such as the Pulmosphere technology from Nektar, or the AIR technology from Alkermese. These prior art methods use combinations of complex and expensive emulsion and spray drying techniques, including significant levels of organic solvents, and producing very low density particles.

High dosing efficiency will have a large number of benefits. For example, as it is possible to repeatedly and reliably deliver a higher proportion of the active agent in a dose, it will be possible to reduce the size of the doses whilst still achieving the same therapeutic effect. Thus, if at present a usual dose of 100µmg of an active agent is used to achieve a desired therapeutic effect and only 10% of the active agent is being properly delivered so that it actually has a therapeutic effect, a dosing efficiency of 70% will allow the dose to be reduced to less than 15µg whilst still achieving the same therapeutic effect! This is clearly very attractive.

Use of the techniques disclosed herein allow high levels of dose reproducibility. The reproducibility is measured in terms of relative standard deviation (RSD%) and is in the order of less than 10, less than 7.5, less than 5, less than 4 or less than 3%. Additionally, the lower dose and the high reproducibility achieved by the present invention means that the therapeutic effect achieved by a given dose will be more predictable and consistent. This obviates the risk of having an unexpected and unusually high dosing efficiency with the conventional devices and powders, which could lead to an undesirably high dose of active agent being administered, effectively an overdose.

Furthermore, high doses of therapeutically active agents has long been linked with the increased incidence of undesirable side effects. Thus, the present invention may help to reduce the incidence of side effects by reducing the dose administered to all patients.

Yet another advantage associated with the higher dosing efficiency of the present invention is that it may be possible to achieve a longer-lasting therapeutic effect without having to increase the dose administered to the patient. The greater dosing efficiency means that a greater amount of a given dose is actually delivered. This can lead to a greater therapeutic effect and, in cases where the active agent does not have a short half-life, this will also mean that the therapeutic effect lasts for a longer period of time. In some circumstances, this may even mean that it is possible to use the present invention to administer an active agent in an immediate release form and achieve the same extended therapeutic effect as a sustained release form of the same active agent.

Naturally, the reduction in the amount of an active agent required to achieve the same therapeutic effect is attractive because of the cost implications. However, it is also likely to be deemed much safer by regulatory bodies such as the FDA in the United States.

Yet another advantage associated with the reduced throat deposition, in that any unpleasant taste effects of the active will be minimised. Also, any side effects such as throat infections caused by deposition of steroids on the throat are reduced.

A particular advantage which is afforded by the high dosing efficiency achieved by the present invention is that it confirms that administration of pharmaceutically active agents in the form of a dry powder and via pulmonary inhalation is an effective and efficient mode of administration. The serum concentration of the active agent following the administration of a dry powder formulation by pulmonary inhalation according to the present invention has been shown to be consistent between doses and between different individuals. There is no variation between individuals, as is observed with other modes of administration (such as oral administration). This means that the therapeutic effect of the administration of a given dose is predictable and reliable. This has the added benefit that a balance can more easily be struck between the therapeutic effect of a pharmaceutically active agent and any adverse effects that might be associated with its administration. This will be demonstrated in one of the examples set out below.

Thus, according to a first aspect of the present invention, a dry powder dispensing device is provided with a pharmaceutical dry powder formulation, as defined in claim 1, wherein the at least 70% of the dose of active agent in the dry powder is administered so as to have a therapeutic effect on the body of a patient. Preferably, the dosing efficiency remains at least 70% over numerous consecutive doses, i.e. the dosing efficiency is reproducible and constant, not an isolated good result.

This high dosing efficiency is achieved by ensuring that each stage of the dose delivery is optimised.

This requires the balancing of various factors which affect the extraction of the powder formulation from the dispensing device, the dynamics of the powder plume created by the device and the deposition of the active particles within the lung. One of the factors affecting these is the tendency of the powder particles to agglomerate. This, in turn, is linked to the size of the particles, as well as other factors, such as the presence of force controlling agents on the surface of the powder particles, particle morphology and density, and the type of device used to dispense the powder. The balancing of such factors is discussed in greater detail below. However, it is clear that the active particles and powder formulations can be tailored to the dispensing device to be used.

It must be appreciated that one cannot focus on just one particular factor affecting dose delivery, to the exclusion of all other factors. This is because the various factors affect one another and the optimisation (if possible) of one factor will not necessarily result in good dosing efficiency without the appropriate adjustment of other factors.

For example, fine particles which do not agglomerate will clearly be beneficial as all of the particles will be of the appropriate size for lung deposition. However, such powder formulations comprising such non-agglomerating particles will have poor flow characteristics, which will make extraction of the powder from the inhaler device difficult, potentially leading to loss of dosing efficiency as a result of increased device retention. If the flowability of the powder is improved, the extraction of the powder from the device is also likely to be improved. However, if the extraction of the powder becomes too easy, this can also have a detrimental effect, which is probably more marked where an active type of dry powder inhaler device is used. As a result of the improved flowability and easier extraction of the powder, it is possible that the powder will actually leave the device too quickly. This can mean that the active particles travel too quickly within the powder plume generated by the device and these particles therefore tend to impact on the subject's throat rather than being inhaled. Thus, the dosing efficiency is once again reduced, this time as a result of increased throat impaction or deposition.

In a preferred embodiment of the present disclosure, the amount of active agent retained in the blister or capsule following actuation of the device is less than 15%, preferably less than 10%, more preferably less than 7% and most preferably less than 5% or 3%.

In another preferred embodiment, the amount of the powder formulation retained in the dispensing device, for example in the blister or capsule, in the mouthpiece and in any vortex chamber or equivalent device part, is less than 15%, preferably less than 10%, more preferably less than 7% and most preferably less than 5% or 3%.

In a yet further embodiment, upon being expelled from the dispensing device, the powder formulation has a dosing efficiency at 5µm of preferably at least 70%, preferably at least 75%, more preferably at least 80%, more preferably at least 85%, more preferably at least 90%, and most preferably at least 95%.

Preferably, upon being expelled from the dispensing device, the powder formulation has a dosing efficiency at 3µm of preferably at least 60%, preferably at least 70%, more preferably at least 75%, more preferably at least 80%, more preferably at least 85%, and most preferably at least 90%.

Preferably, upon being expelled from the dispensing device, the powder formulation has a dosing efficiency at 2µm of preferably at least 40%, preferably at least 50%, more preferably at least 55%, more preferably at least 60%, and most preferably at least 70%. These efficiencies are far greater than anything consistently achieved prior to this invention.

In another preferred embodiment, the particles comprising a pharmaceutically active agent (active particles) have a mass median aerodynamic diameter (MMAD) of less than 10µm. Preferably the MMAD of the active particles is less than 7µm, more preferably less than 5µm, more preferably less than 2µm, and most preferably less than 1.5µm.

Finally, in another preferred embodiment, the amount of the active agent which is deposited in the throat of the user is less than 15% of the active agent in the metered dose. Preferably, throat deposition is less than 10%, more preferably it is less than 7% and most preferably it is less than 5% or less than 3%.

The foregoing powder retention, FPF, MMAD and throat deposition figures may be achieved by adopting one or more of the following adaptations to conventional dry powder dispensing devices, dry powder formulations or methods for preparing dry powder formulations. Combinations of these will lead to a dose delivery of at least 70%.

Preferred embodiments disclosed herein will now be described in detail in the following sections of this specification. These embodiments represent various separate means of putting the present disclosure into effect. These embodiments may be used separately or in combination. When used in combination, the embodiments described in the following sections will provide enhanced results in terms of dosing efficiency and dose reproducibility.

Reproducibility is very important in the present invention. The unpredictable nature of the conventional powder systems means that doses they provide can vary significantly. Given that the dosing is usually inefficient, the amount of active agent in a dose is generally much higher than is to actually be administered to the subject. However, the variable efficiency of the dosing can result in too much active agent being administered and this may be the cause of adverse side effects in some instances. Alternatively, the dosing may be less efficient than predicted, leading to an ineffective dose being administered so that the desired therapeutic effect is not achieved.

Where the dosing is unpredictable, it is possible for conventional powder systems to provide high dosing efficiency on a one-off basis. However, these conventional powder systems will not provide high dosing efficiency on a consistent or repeatable and predictable basis, as the powder systems according to the present invention do.

The present invention can be carried out with any pharmaceutically active agent. The preferred active agents include:
1) steroid drugs such as, for example, alcometasone, beclomethasone, beclomethasone dipropionate, betamethasone, budesonide, clobetasol, deflazacort, diflucortolone, desoxymethasone, dexamethasone, fludrocortisone, flunisolide, fluocinolone, fluometholone, fluticasone, fluticasone proprionate, hydrocortisone, triamcinolone, nandrolone decanoate, neomycin sulphate, rimexolone, methylprednisolone and prednisolone;
2) antibiotic and antibacterial agents such as, for example, metronidazole, sulphadiazine, triclosan, neomycin, amoxicillin, amphotericin, clindamycin, aclarubicin, dactinomycin, nystatin, mupirocin and chlorhexidine;
3) systemically active drugs such as, for example, isosorbide dinitrate, isosorbide mononitrate, apomorphine and nicotine;
4) antihistamines such as, for example, azelastine, chlorpheniramine, astemizole, cetirizine, cinnarizine, desloratadine, loratadine, hydroxyzine, diphenhydramine, fexofenadine, ketotifen, promethazine, trimeprazine and terfenadine;
5) anti-inflammatory agents such as, for example, piroxicam, nedocromil, benzydamine, diclofenac sodium, ketoprofen, ibuprofen, heparinoid, nedocromil, cromoglycate, fasafungine and iodoxamide;
6) anticholinergic agents such as, for example, atropine, benzatropine, biperiden, cyclopentolate, oxybutinin, orphenadine hydrochloride, glycopyrronium, glycopyrrolate, procyclidine, propantheline, propiverine, tiotropium, tropicamide, trospium, ipratropium bromide and oxitroprium bromide;
7) anti-emetics such as, for example, bestahistine, dolasetron, nabilone, prochlorperazine, ondansetron, trifluoperazine, tropisetron, domperidone, hyoscine, cinnarizine, metoclopramide, cyclizine, dimenhydrinate and promethazine;
8) hormonal drugs such as, for example, protirelin, thyroxine, salcotonin, somatropin, tetracosactide, vasopressin or desmopressin;
9) bronchodilators, such as salbutamol, fenoterol and salmeterol;
10) sympathomimetic drugs, such as adrenaline, noradrenaline, dexamfetamine, dipirefin, dobutamine, dopexamine, phenylephrine, isoprenaline, dopamine, pseudoephedrine, tramazoline and xylometazoline;
11) anti-fungal drugs such as, for example, amphotericin, caspofungin, clotrimazole, econazole nitrate, fluconazole, ketoconazole, nystatin, itraconazole, terbinafine, voriconazole and miconazole;
12) local anaesthetics such as, for example, amethocaine, bupivacaine, hydrocortisone, methylprednisolone, prilocaine, proxymetacaine, ropivacaine, tyrothricin, benzocaine and lignocaine;
13) opiates, preferably for pain management, such as, for example, buprenorphine, dextromoramide, diamorphine, codeine phosphate, dextropropoxyphene, dihydrocodeine, papaveretum, pholcodeine, loperamide, fentanyl, methadone, morphine, oxycodone, phenazocine, pethidine and combinations thereof with an anti-emetic;
14) analgesics and drugs for treating migraine such as clonidine, codine, coproxamol, dextropropoxypene, ergotamine, sumatriptan, tramadol and non-steroidal anti-inflammatory drugs;
15) narcotic agonists and opiate antidotes such as naloxone, and pentazocine;
16) phosphodiesterase type 5 inhibitors, such as sildenafil; and
17) pharmaceutically acceptable salts of any of the foregoing.

A plurality of active agents can be employed in the practice of the present invention.

In preferred embodiments, the active agent is heparin, apomorphine, glycopyrrolate, clomipramine or clobozam.

### Delivery Devices

The device used to deliver the dry powder formulations is clearly going to affect the performance of the dry powder formulations and the device is therefore a very important part of present invention.

Dry powder inhaler devices (DPIs) are well known in the art and there are a variety of different types. Generally, the dry powder is stored within the device and is extracted from the place of storage upon actuation of the device, whereupon the powder is expelled from the device in the form of a plume of powder which is to be inhaled by the subject. In most DPIs, the powder is stored in a unitary manner, for example in blisters or capsules containing a predetermined amount of the dry powder formulation. Some DPIs have a powder reservoir and doses of the powder are measured out within the device. These reservoir devices are less favoured in the present invention as the blisters or capsules tend to provide more accurate doses.

As briefly discussed above, there are a number of factors associated with the delivery devices which will affect the dosing efficiency achieved. Firstly, there is the extraction of the dose. Additionally, the dynamics of the powder plume generated will also affect dosing delivery.

The dry powder inhaler devices suitable for use in the present invention include "single dose" devices, for example the Rotahaler (trade mark), the Spinhaler (trade mark) and the Diskhaler (trade mark) in which individual doses of the powder composition are introduced into the device in, for example, single dose capsules or blisters, and also multiple dose devices, for example the Turbohaler (trade mark) in which, on actuation of the inhaler, one dose of the powder is removed from a reservoir of the powder material contained in the device.

Dry powder inhalers can be "passive" devices in which the patient's breath is the only source of gas which provides a motive force in the device. Examples of "passive" dry powder inhaler devices include the Rotahaler and Diskhaler (GlaxoSmithKline) and the Turbohaler (Astra-Draco) and Novolizer (trade mark) (Viatris GmbH). Alternatively, "active" devices may be used, in which a source of compressed gas or alternative energy source is used. Examples of suitable active devices include Aspirair (trade mark) (Vectura Ltd) and the active inhaler device produced by Nektar Therapeutics.

Particularly preferred "active" dry powder inhalers are described in more detail in WO 01/00262, WO 02/07805, WO 02/89880 and WO 02/89881. It should be appreciated, however, that the compositions disclosed herein can be administered with either passive or active inhaler devices.

According to an embodiment of the present invention, an active inhaler device may be used to dispense the apomorphine dry powder formulations, in order to ensure that the best fine particle fraction and fine particle dose is achieved and, very importantly, that this is achieved consistently. Preferably, the inhaler device includes a breath triggering means such that the delivery of the dose is triggered by the onset of the patient's inhalation. This means that the patient does not need to coordinate their inhalation with the actuation of the inhaler device and that the dose can be delivered at the optimum point in the inspiratory flow. Such devices are commonly referred to as "breath actuated".

As already mentioned, in the case of certain powders, an active inhaler device offers advantages in that a higher fine particle fraction and a more consistent dose to dose repeatability will be obtainable than if other forms of device were used. Such devices include, for example, the Aspirair (trade mark) or the Nektar Therapeutics active inhaler device, and may be breath actuated devices of the kind in which generation of an aerosolised cloud of powder is driven by inhalation of the patient.

### Dose Extraction

It is common for dry powder formulations to be pre-packaged in individual doses, usually in the form of capsules or blisters which each contain a single dose of the powder. In such devices, the doses will be accurately measured and consistent.

However, it is also known for powders to be held in a reservoir in a dispensing device. In such a case, a predetermined amount of powder is measured out and then dispensed by the device. Inevitably, such an arrangement will allow for some variation in the size of the dose between actuations of the same device. This will especially be the case where the amount of powder to be dispensed is relatively small, as it is difficult to accurately measure out small amounts of dry powder in such devices. Therefore, as the present invention is concerned with dose accuracy and reproducibility, devices which hold the dry powder to be dispensed in a reservoir are not preferred.

Actuation of the dispensing device refers to the process during which a dose of the dry powder formulation is removed from its rest position in the inhaler (be it in a blister or capsule or other container). The actuation may be caused by the user of the device inhaling in the case of a passive device, or by firing an active device. The actuation of a dispensing device occurs after the powder has been loaded ready for use within the device.

### Improved Evacuation of Dose from Packaging

As already mentioned above, it is common for some of the dose to be deposited in the inhaler when it is used or, for some of the dose to remain in the pack in which the dose is stored. Reference will now be made to embodiments of the disclosure which seek to minimise the deposition of the dose on the inhaler and the retention of dose within the pack.

It will be appreciated that an important factor in maintaining the efficiency, accuracy and repeatability of the dose is to minimise the amount of drug that is retained in the inhaler mechanism and in the medicament pack in which the drug is stored prior to inhalation using the device. A conventional pack for an individual dose of dry powder medicament may include a gelatin capsule or a foil blister which is cold formed from a ductile foil laminate. A piercable foil laminate lid usually covers the blister which is heat sealed around the periphery of the blister. These types of package are preferred because each dose is protected from the ingress of water and penetration of gases such as oxygen in addition to being shielded from light and UV radiation and so offer excellent environmental protection. To administer a dose using a compressed gas powered inhaler, the capsule or foil lid is punctured by a piercing mechanism so that the drug can be entrained and carried to an aerosolising means, such as a nozzle, in a charge of gas which passes through the capsule or blister to the nozzle.

In an active inhaler of the aforementioned type, the same charge of gas provides the energy needed for both entraining the drug to evacuate the packaging and for aerosolising the drug once it has reached the nozzle. It is therefore important that the primary packaging does not present a significant restriction to the gas flow from the source of pressurised gas to the aerosolising nozzle. Bearing in mind that the amount of gas available for each dose is limited by what can be stored in a pressurised canister or generated in the device by the user by, for example, using a manually operated pump, the efficiency by which the drug is entrained in the airflow and so evacuated from its packaging must be as high as possible.

As mentioned above, a problem with known inhalation devices is that it is possible for not all of the drug to be entrained in the airflow each time the device is used because the blister or capsule, in which the dose is stored, is typically pierced in such a way that the gas flowing into the blister through the pierced foil only partially scours the blister surfaces before flowing out of the blister. This problem is often exacerbated by the flap of foil cut by the piercing element as this can obscure parts of the blister from the flow of gas thereby restricting the free flow of gas throughout the entire volume of the blister and creating "dead" regions where gas flow is minimal or where secondary eddies form leading to powder becoming trapped. This trapped powder will have a significant detrimental effect on the repeatablility and accuracy of the delivered dose as well as on the overall efficiency of the inhaler.

This aspect of the disclosure seeks to provide a dry powder inhaler in which all, or substantially all, of the internal surfaces of a pack containing a medicament dose are swept by the airflow so that substantially all of the drug is evacuated from the pack for delivery through an aerosolising nozzle and out of the device into the airway of a patient, thereby improving the delivered dose and hence the fine particle fraction of the delivered dose.

### Particle Cohesiveness

For formulations to reach the deep lung or the blood stream via inhalation, the active agent in the formulation must be in the form of very fine particles, for example, having a mass median aerodynamic diameter (MMAD) of less than 10µm. It is well established that particles having an MMAD of greater than 10µm are likely to impact on the walls of the throat and generally do not reach the lung. Particles having an MMAD in the region of 5µm to 2µm will generally be deposited in the respiratory bronchioles whereas particles having an MMAD in the range of 3 to 0.05µm are likely to be deposited in the alveoli or be absorbed into the bloodstream.

Preferably, for delivery to the lower respiratory tract or deep lung, the MMAD of the active particles is not more than 10µm, and preferably not more than 5µm, more preferably not more than 3µm, and may be less than 1µm. Ideally, at least 90% by weight of the active particles in a dry powder formulation should have an MMAD of not more than 10µm, preferably not more than 5µm, more preferably not more than 3µm and most preferably not more than 1µm.

When dry powders are produced using conventional processes, the active particles will vary in size, and often this variation can be considerable. This can make it difficult to ensure that a high enough proportion of the active particles are of the appropriate size for administration to the correct site. It is therefore desirable to have a dry powder formulation wherein the size distribution of the active particles is as narrow as possible. This will improve dose efficiency and reproducibility.

Fine particles, that is, those with an MMAD of less than 10µm, are thermodynamically unstable due to their high surface area to volume ratio, which provides a significant excess surface free energy and encourages the particles to agglomerate. In the inhaler, agglomeration of fine particles and adherence of such particles to the walls of the inhaler are problems that result in the fine particles leaving the inhaler as large, stable agglomerates, or being unable to leave the inhaler and remaining adhered to the interior of the inhaler, or even clogging or blocking the inhaler.

The uncertainty as to the extent of formation of stable agglomerates of the particles between each actuation of the inhaler, and also between different inhalers and different batches of particles, leads to poor dose reproducibility. Furthermore, the formation of agglomerates means that the MMAD of the active particles can be vastly increased, with agglomerates of the active particles not reaching the required part of the lung.

The tendency of fine particles to agglomerate means that the FPF of a given dose is highly unpredictable and a variable proportion of the fine particles will be administered to the lung, or to the correct part of the lung, as a result.

In an attempt to improve this situation and to provide a consistent FPF and FPD, dry powder formulations often include additive material.

The additive material is intended to decrease the cohesion between particles in the dry powder formulation. It is thought that the additive material interferes with the weak bonding forces between the small particles, helping to keep the particles separated and reducing the adhesion of such particles to one another, to other particles in the formulation if present and to the internal surfaces of the inhaler device. Where agglomerates of particles are formed, the addition of particles of additive material decreases the stability of those agglomerates so that they are more likely to break up in the turbulent air stream created on actuation of the inhaler device, whereupon the particles are expelled from the device and inhaled. As the agglomerates break up, the active particles return to the form of small individual particles which are capable of reaching the lower lung.

In the prior art, dry powder formulations are discussed which include distinct particles of additive material (generally of a size comparable to that of the fine active particles). In some embodiments, the additive material may form a coating, generally a discontinuous coating, on the active particles and/or any carrier particles.

Preferably, the additive material is an anti-adherent material and it will tend to reduce the cohesion between particles and will also prevent fine particles becoming attached to the inner surfaces of the inhaler device. Advantageously, the additive material is an anti-friction agent or glidant and will give better flow of the pharmaceutical composition in the inhaler. The additive materials used in this way may not necessarily be usually referred to as anti-adherents or anti-friction agents, but they will have the effect of decreasing the cohesion between the particles or improving the flow of the powder. The additive materials are often referred to as force control agents (FCAs) and they usually lead to better dose reproducibility and higher fine particle fractions.

Therefore, an FCA, as used herein, is an agent whose presence on the surface of a particle can modify the adhesive and cohesive surface forces experienced by that particle, in the presence of other particles. In general, its function is to reduce both the adhesive and cohesive forces.

In general, the optimum amount of additive material to be included in a dry powder formulation will depend on the chemical composition and other properties of the additive material and of the active material, as well as upon the nature of other particles such as carrier particles, if present. In general, the efficacy of the additive material is measured in terms of the fine particle fraction of the composition.

Known additive materials usually consist of physiologically acceptable material, although the additive material may not always reach the lung, for example where the additive particles are attached to the surface of carrier particles so that they will generally be deposited, along with those carrier particles, at the back of the throat of the user.

Preferred additive materials for used in prior art dry powder formulations include amino acids, peptides and polypeptides having a molecular weight of between 0.25 and 1000 kDa and derivatives thereof, dipolar ions such as zwitterions, phospholipids such as lecithin, and metal stearates such as magnesium stearate.

In a further attempt to improve this situation and to provide a consistent FPF and FPD, dry powder formulations often include coarse carrier particles of excipient material mixed with fine particles of active material. Rather that sticking to one another, the fine active particles tend to adhere to the surfaces of the coarse carrier particles whilst in the inhaler device, but are supposed to release and become dispersed upon actuation of the dispensing device and inhalation into the respiratory tract, to give a fine suspension. The carrier particles preferably have MMADs greater than 90µm.

The inclusion of coarse carrier particles is also very attractive where very small doses of active agent are dispensed. It is very difficult to accurately and reproducibly dispense very small quantities of powder and small variations in the amount of powder dispensed will mean large variations in the dose of active agent where the powder comprises mainly active particles. Therefore, the addition of a diluent, in the form of large excipient particles will make dosing more reproducible and accurate.

Carrier particles may be of any acceptable excipient material or combination of materials. For example, the carrier particles may be composed of one or more materials selected from sugar alcohols, polyols and crystalline sugars. Other suitable carriers include inorganic salts such as sodium chloride and calcium carbonate, organic salts such as sodium lactate and other organic compounds such as polysaccharides and oligosaccharides. Advantageously the carrier particles are of a polyol. In particular the carrier particles may be particles of crystalline sugar, for example mannitol, dextrose or lactose. Preferably, the carrier particles are of lactose.

Advantageously, substantially all (by weight) of the carrier particles have a diameter which lies between 20µm and 1000µm, more preferably 50µm and 1000µm. Preferably, the diameter of substantially all (by weight) of the carrier particles is less than 355µm and lies between 20µm and 250µm.

Preferably at least 90% by weight of the carrier particles have a diameter between from 60µm to 180µm. The relatively large diameter of the carrier particles improves the opportunity for other, smaller particles to become attached to the surfaces of the carrier particles and to provide good flow and entrainment characteristics and improved release of the active particles in the airways to increase deposition of the active particles in the lower lung.

The ratios in which the carrier particles (if present) and composite active particles are mixed will, of course, depend on the type of inhaler device used, the type of active particles used and the required dose. The carrier particles may be present in an amount of at least 50%, more preferably 70%, advantageously 90% and most preferably 95% based on the combined weight of the composite active particles and the carrier particles.

However, a further difficulty is encountered when adding coarse carrier particles to a composition of fine active particles and that difficulty is ensuring that the fine particles detach from the surface of the large particles upon actuation of the delivery device.

The step of dispersing the active particles from other active particles and from carrier particles, if present, to form an aerosol of fine active particles for inhalation is significant in determining the proportion of the dose of active material which reaches the desired site of absorption in the lungs. In order to improve the efficiency of that dispersal it is known to include in the composition additive materials of the nature discussed above. Compositions comprising fine active particles and additive materials are disclosed in WO 97/03649 and WO 96/23485.

In light of the foregoing problems associated with known dry powder formulations, even when including additive material and/or carrier particles, it is an aim of the present invention to provide dry powder compositions which have physical and chemical properties which lead to an enhanced FPF and FPD. This leads to greater dosing efficiency, with a greater proportion of the dispensed active agent reaching the desired part of the lung for achieving the required therapeutic effect.

It is highly desirable to be able to prepare fine particles comprising an active agent using simple methods and simple apparatus. As discussed below, dry powder formulations can be prepared, without requiring elaborate, multi-step methods, wherein the active particle have an MMAD suitable for deposition in the deep lung and wherein the dry powder formulations exhibit the preferred FPF and FPD discussed above, regardless of the type of device used to dispense them.

Known additive materials or force control agents (FCAs) usually consist of physiologically acceptable material, although the FCAs may not always reach the lung. For example, where additive particles are attached to the surface of carrier particles, they will generally be deposited, along with those carrier particles, at the back of the throat of the user.

Advantageously, the FCA includes one or more compounds selected from amino acids and derivatives thereof, and peptides and derivatives thereof. Amino acids, peptides and derivatives of peptides are physiologically acceptable and give acceptable release of the active particles on inhalation.

It is particularly advantageous for the FCA to comprise an amino acid. The FCA may comprise one or more of any of the following amino acids: leucine, isoleucine, lysine, valine, methionine, and phenylalanine. The FCA may be a salt or a derivative of an amino acid, for example aspartame or acesulfame K. Preferably, the FCA consists substantially of an amino acid, more preferably of leucine, advantageously L-leucine. The D-and DL-forms may also be used. As indicated above, leucine has been found to give particularly efficient dispersal of the active particles on inhalation.

The FCA may include one or more water soluble substances. This helps absorption of the FCA by the body if it reaches the lower lung. The FCA may include dipolar ions, which may be zwitterions. It is also advantageous to include a spreading agent as an FCA, to assist with the dispersal of the composition in the lungs. Suitable spreading agents include surfactants such as known lung surfactants (e.g. ALEC, Registered Trade Mark) which comprise phospholipids, for example, mixtures of DPPC (dipalmitoyl phosphatidylcholine) and PG (phosphatidylglycerol). Other suitable surfactants include, for example, dipalmitoyl phosphatidylethanolamine (DPPE), dipalmitoyl phosphatidylinositol (DPPI).

The FCA may comprise a metal stearate, or a derivative thereof, for example, sodium stearyl fumarate or sodium stearyl lactylate. Advantageously, it comprises a metal stearate. For example, zinc stearate, magnesium stearate, calcium stearate, sodium stearate or lithium stearate. Preferably, the additive material comprises magnesium stearate.

The FCA may include or consist of one or more surface active materials, in particular materials that are surface active in the solid state, which may be water soluble or water dispersible, for example lecithin, in particular soya lecithin, or substantially water insoluble, for example solid state fatty acids such as oleic acid, lauric acid, palmitic acid, stearic acid, erucic acid, behenic acid, or derivatives (such as esters and salts) thereof such as glyceryl behenate. Specific examples of such materials are phosphatidylcholines, phosphatidylethanolamines, phosphatidylglycerols and other examples of natural and synthetic lung surfactants; lauric acid and its salts, for example, sodium lauryl sulphate, magnesium lauryl sulphate; triglycerides such as Dynsan 118 and Cutina HR; and sugar esters in general. Alternatively, the FCA may be cholesterol.

Other possible FCAs include sodium benzoate, hydrogenated oils which are solid at room temperature, talc, titanium dioxide, aluminium dioxide, silicon dioxide and starch. Also useful as FCAs are film-forming agents, fatty acids and their derivatives, as well as lipids and lipid-like materials.

In one embodiment disclosed herein, the FCA comprises an amino acid, a derivative of an amino acid, or a phospholipid. Preferably, the FCA comprises one or more of L-, D- or DL- forms of leucine, isoleucine, lysine, valine, methionine, phenylalanine, or Aerocine, lecithin. Disclosed herein, the FCA comprises leucine and preferably 1-leucine. The FCA of the present invention is a metal stearate, preferably magnesium stearate.

In some embodiments, a plurality of different FCAs can be used.

As touched upon above, for the best powder performance, the powder formulations disclosed herein need to exhibit particle cohesiveness which is tailored to the type of device being used to dispense it. Where the device is efficient at extracting the powder from the device, such as is this case with active dispensing devices such as Aspirair (trade mark), the powder formulation preferably exhibits a degree of cohesiveness in order to retard the expulsion of the powder from the device. This, in turn, has a beneficial effect on the plume dynamics, leading to reduced deposition of the powder in the throat.

The discussions below look at different approaches to particle engineering, allowing one to control and refine the particle cohesion, so that ideal powder behaviour and performance can be achieved and this can be matched to the device to be used to dispense the powder.

### Micronised Dry Powder Particles

In another aspect disclosed herein, a method of producing powders is provided wherein the method achieves a further reduction in the size of the active particles, preferably so that the particles are of an appropriate size for administration to the deep lung by inhalation. Preferably, this is possible using both active dry powder inhaler devices and passive dry powder inhaler devices.

In particular, the present invention seeks to optimise the preparation of particles of active agent used in the dry powder composition by engineering the particles making up the dry powder composition and, in particular, by engineering the particles of active agent. It is proposed to do this by adjusting and adapting the milling process used to form the particles of active agent.

According to an aspect of the present disclosure, a method is provided for making composite active particles for use in a pharmaceutical composition for pulmonary inhalation, the method comprising jet milling active particles in the presence of additive material, preferably wherein the jet milling is conducted using air or a compressible gas or fluid.

In the conventional use of the word, "milling" means the use of any mechanical process which applies sufficient force to the particles of active material that it is capable of breaking coarse particles (for example, particles with a MMAD greater than 100µm) down to fine particles (for example, having a MMAD not more than 50µm). In the present invention, the term "milling" also refers to deagglomeration of particles in a formulation, with or without particle size reduction. The particles being milled may be large or fine prior to the milling step.

In the prior art, co-milling or co-micronising active agents and additive materials have been suggested. It is stated that milling can be used to substantially decrease the size of particles of active agent. However, if the particles of active agent are already fine, for example have a MMAD of less than 20µm prior to the milling step, the size of those particles may not be significantly reduced where the milling of these active particles takes place in the presence of an additive material. Rather, milling of fine active particles with additive particles using the methods described in the prior art (for example, in WO 02/43701) will result in the additive material becoming deformed and being smeared over or fused to the surfaces of the active particles. The resultant composite active particles have been found to be less cohesive after the milling treatment. However, there is still the disadvantage that this is not combined with a significant reduction in the size of the particles.

The prior art mentions two types of processes in the context of co-milling or co-micronising active and additive particles.

First, there is the compressive type process, such as Mechano-Fusion and Cyclomix methods. As the name suggests, Mechano-Fusion is a dry coating process designed to mechanically fuse a first material onto a second material. The first material is generally smaller and/or softer than the second. The Mechano-Fusion and Cyclomix working principles are distinct from alternative milling techniques in having a particular interaction between an inner element and a vessel wall, and are based on providing energy by a controlled and substantial compressive force.

The fine active particles and the additive particles are fed into the Mechano-Fusion driven vessel (such as a Mechano-Fusion system (Hosokawa Micron Ltd)), where they are subject to a centrifugal force and are pressed against the vessel inner wall. The powder is compressed between the fixed clearance of the drum wall and a curved inner element with high relative speed between drum and clement. The inner wall and the curved element together form a gap or nip in which the particles are pressed together. As a result, the particles experience very high shear forces and very strong compressive stresses as they are trapped between the inner drum wall and the inner element (which has a greater curvature than the inner drum wall). The particles are pressed against each other with enough energy to locally heat and soften, break, distort, flatten and wrap the additive particles around the core particle to form a coating. The energy is generally sufficient to break up agglomerates and some degree of size reduction of both components may occur.

These Mechano-Fusion and Cyclomix processes apply a high enough degree of force to separate the individual particles of active material and to break up tightly bound agglomerates of the active particles such that effective mixing and effective application of the additive material to the surfaces of those particles is achieved. An especially desirable aspect of the described co-milling processes is that the additive material becomes deformed in the milling and may be smeared over or fused to the surfaces of the active particles.

However, in practice, this compression process produces little or no milling (i.e. size reduction) of the drug particles, especially where they are already in a micronised form (i.e. <10µm), the only physical change which may be observed is a plastic deformation of the particles to a rounder shape.

Secondly, there are the impact milling processes involved in ball milling and the use of a homogenizer.

Ball milling is a suitable milling method for use in the prior art co-milling processes. Centrifugal and planetary ball milling are especially preferred methods. Alternatively, a high pressure homogeniser may be used in which a fluid containing the particles is forced through a valve at high pressure producing conditions of high shear and turbulence. Such homogenisers may be more suitable than ball mills for use in large scale preparations of the composite active particles.

Suitable homogensiers include EmulsiFlex high pressure homogenisers which are capable of pressures up to 4000 bar, Niro Soavi high pressure homogenisers (capable of pressures up to 2000 bar), and Microfluidics Microfluidisers (maximum pressure 2750 bar). The milling step may, alternatively, involve a high energy media mill or an agitator bead mill, for example, the Netzsch high energy media mill, or the DYNO-mill (Willy A. Bachofen AG, Switzerland).

These processes create high-energy impacts between media and particles or between particles. In practice, while these processes are good at making very small particles, it has been found that neither the ball mill nor the homogenizer was effective in producing dispersion improvements in resultant drug powders in the way observed for the compressive process. It is believed that the second impact processes are not as effective in producing a coating of additive material on each particle.

Conventional methods comprising co-milling active material with additive materials (as described in WO 02/43701) result in composite active particles which are fine particles of active material with an amount of the additive material on their surfaces. The additive material is preferably in the form of a coating on the surfaces of the particles of active material. The coating may be a discontinuous coating. The additive material may be in the form of particles adhering to the surfaces of the particles of active material.

At least some of the composite active particles may be in the form of agglomerates. However, when the composite active particles are included in a pharmaceutical composition, the additive material promotes the dispersal of the composite active particles on administration of that composition to a patient, via actuation of an inhaler.

Jet mills are capable of reducing solids to particle sizes in the low-micron to submicron range. The grinding energy is created by gas streams from horizontal grinding air nozzles. Particles in the fluidized bed created by the gas streams are accelerated towards the centre of the mill, colliding with slower moving particles. The gas streams and the particles carried in them create a violent turbulence and as the particles collide with one another they are pulverized.

In the past, jet-milling has not been considered attractive for co-milling active and additive particles, processes like Mechano-Fusion and Cyclomixing being clearly preferred. The collisions between the particles in a jet mill are somewhat uncontrolled and those skilled in the art, therefore, considered it unlikely for this technique to be able to provide the desired deposition of a coating of additive material on the surface of the active particles. Moreover, it was believed that, unlike the situation with Mechano-Fusion and Cyclomixing, segregation of the powder constituents occurred in jet mills, such that the finer particles, that were believed to be the most effective, could escape from the process. In contrast, it could be clearly envisaged how techniques such as Mechano-Fusion would result in the desired coating.

It should also be noted that it was also previously believed that the compressive or impact milling processes must be carried out in a closed system, in order to prevent segregation of the different particles. This has also been found to be untrue and the co-jet milling processes according to the present invention do not need to be carried out in a closed system. Even in an open system, the co-jet milling has surprisingly been found not to result in the loss of the small particles, even when using leucine as the additive material.

It has now unexpectedly been discovered that composite particles of active and additive material can be produced by co-jet milling these materials. The resultant particles have excellent characteristics which lead to greatly improved performance when the particles are dispensed from a DPI for administration by inhalation. In particular, co-jet milling active and additive particles can lead to further significant particle size reduction. What is more, the composite active particles exhibit an enhanced FPD and FPF, compared to those disclosed in the prior art.

The effectiveness of the promotion of dispersal of active particles has been found to be enhanced by using the co-jet milling methods according to the present invention in comparison to compositions which are made by simple blending of similarly sized particles of active material with additive material. The phrase "simple blending" means blending or mixing using conventional tumble blenders or high shear mixing and basically the use of traditional mixing apparatus which would be available to the skilled person in a standard laboratory.

It has been found that, contrary to previous belief, co-jet milling can be used to produce sufficiently complete coatings of additive material, which have now been observed to substantially improve the dispersion of the powders from an inhaler. The jet milling process can also be adjusted to tailor the composite particles to the type of inhaler device to be used to dispense the particles. The inhaler device may be an active inhaler device, such as Aspirair (trade mark) or it may be a passive device.

Further, the co-jet milling process may optionally also be arranged so as to significantly mill the active particles, that is, to significantly reduce the size of the active particles. The co-jet milling of the present invention may even, in certain circumstances, be more efficient in the presence of the additive material than it is in the absence of the additive material. The benefits are that it is therefore possible to produce smaller particles for the same mill, and it is possible to produce milled particles with less energy. Co-jet milling should also reduce the problem of amorphous content by both creating less amorphous material, as well as hiding it below a layer of additive material.

The impact forces of the co-jet milling are sufficient to break up agglomerates of drug, even micronised drug, and are effective at distributing the additive material to the consequently exposed faces of the particles. This is an important aspect of the present invention. It has been shown that if the energy of the process is not sufficient to break up the agglomerates of drug (for example, as will be the case when one uses a conventional blender), the additive material merely coats the agglomerates and these agglomerates can even be compressed, making them even more difficult to disperse. This is clearly undesirable when one is seeking to prepare a dry powder for administration by inhalation.

Fine particles of active material suitable for pulmonary administration have often been prepared by milling in the past. However, when using many of the known milling techniques, once the particles reach a minimum size, referred to as the "critical size", they tend to re-combine at the same rate as being fractured, or do not fracture effectively and therefore no further reduction in the particle size is achieved. Critical sizes are specific to particular mills and sets of milling conditions.

Thus, manufacture of fine particles by milling can require much effort and there are factors which consequently place limits on the minimum size of particles of active material which can be achieved, in practice, by such milling processes.

The present invention consequently relates to the provision of a high-energy impact process that is effective in producing improvements in the resultant drug powders.

Furthermore, contrary to conventional thinking, the processes disclosed herein do not need to be carried out in a closed system. Even where the additive material being co-jet milled is leucine, there is no observed loss of additive material or reduction in coating where the jet-milling is not carried out in a closed system. Rather, in one embodiment disclosed herein, the method disclosed herein is carried out in a flow-through system, without any loss in performance of the resultant composite particles. This is an economically important feature, as it can significantly increase the rate of production of the powders of the invention.

In one embodiment of the present disclosure, 90% by mass of the active particles jet-milled are initially less than 20µm in diameter. More preferably, 90% by mass of the active particles jet-milled are initially less than 10µm in diameter, and most preferably less than 5µm in diameter.

In another embodiment, 90% by mass of the additive particles jet-milled are initially less than 20µm in diameter. More preferably, 90% by mass of the additive particles jet-milled are initially less than 10µm in diameter, and most preferably less than 5µm in diameter or less than 3µm in diameter.

The terms "active particles" and "particles of active material" and the like are used interchangeably herein. The active particles comprise one or more pharmaceutically active agents.

Preferably, the active agent is a small molecule, as opposed to a macromolecule. Preferably, the active agent is not a protein, and more preferably, the active agent is not insulin. In the case of proteins and in particular insulin, there is little or no benefit to be derived from the use of a force control agent in a dry powder formulation for administration by inhalation. The reason for this is that in the case of these active agents, the active agent itself acts as a force control agent and the cohesive forces of particles of these active agents are already only weak.

In preferred embodiments of the present invention, the active agent is heparin, apomorphine, clobozam, clomipramine or glycopyrrolate.

The terms "additive particles" and "particles of additive material" are used interchangeably herein. The additive particles comprise one or more additive materials (or FCAs). Preferably, the additive particles consist essentially of the additive material.

Suitable additive materials for use in the milling methods disclosed herein are listed above (as FCAs).

In general, the optimum amount of additive material to be included in a dry powder formulation will depend on the chemical composition and other properties of the additive material and of the active material, as well as upon the nature of other particles, such as carrier particles, if present. In general, the efficacy of the additive material is measured in terms of the FPF of the composition.

In one embodiment of the present invention, composite active particles produced by co-jet milling according to the present invention are mixed with carrier particles made of an inert excipient material.

Where the powder composition comprises an active material, additive material and excipient material, this is referred to as a 3-component system. In contrast, a 2-component system comprises just active and additive materials.

Excipient materials may be included in powders for administration by pulmonary inhalation for a number of reasons. On the one hand, the inclusion of particles of excipient material of an appropriate size can enhance the flow properties of the powder and can enhance the powder's handleability. Excipient material is also added to powder formulations as a diluent. It can be very difficult to accurately and reproducibly administer a very small amount of powder. Where low doses of drug are required, this can pose a problem and so it can be desirable to add a diluent to the powder, to increase the amount of powder to be dispensed.

In one embodiment of the present invention, the excipient material is in the form of relatively large or coarse carrier particles. Advantageously, substantially all (by weight) of the carrier particles have a diameter which lies between about 20µm and about 1000µm, more preferably about 50µm and about 1000µm. Preferably, the diameter of substantially all (by weight) of the carrier particles is less than about 355µm and lies between about 20µm and about 250µm.

Preferably at least about 90% by weight of the carrier particles have a diameter between from about 60µm to about 180µm. The relatively large diameter of the carrier particles improves the opportunity for other, smaller particles to become attached to the surfaces of the carrier particles and provides good flow and entrainment characteristics and improved release of the active particles in the airways to increase deposition of the active particles in the lung.

Conventional thinking regarding carrier particles is that they improve the poor flowability of formulations comprising fine particles of less than 10µm. The poor flowability is due to the agglomeration of the fine particles which occurs due to the strong attractive forces between the small particles. In the presence of large carrier particles, these attractive forces cause the fine particles to become attached to the surface of the large carrier particles, forming (usually discontinuous) coatings. This arrangement of the large and fine particles leads to better flow characteristics than is observed with a formulation made up solely of fine active particles.

The carrier particles to be added to the composite active particles of the present invention are relatively large particles of an excipient material, such as lactose.

The ratios in which the carrier particles and composite active particles are mixed will, of course, depend on the type of inhaler device used, the type of active particles used and the required dose. The carrier particles may be present in an amount of at least about 50%, more preferably at least about 70%, more preferably at least about 80%, advantageously at least about 90% and most preferably at least about 95%, based on the combined weight of the composite active particles and the carrier particles.

A 3-component system including carrier particles, such as the one described above, would be expected to work well in a passive device. The presence of the carrier particles makes the powder easier to extract from the blister, capsule or other storage means. The powder extraction tends to pose more of a problem in passive devices, as they do not create as turbulent an air flow through the blister upon actuation as active devices. This means that it can be difficult to entrain all of the powder in the air flow. The powder entrainment in a passive device is made easier where the powder includes carrier particles as this will mean that the powder is less cohesive and exhibits better flowability, compared with a powder consisting entirely of smaller particles, for example all having a diameter of less than 10µm.

The composite active particles of the invention should readily release from the surface of the carrier particles of the invention upon actuation of the dispensing device by virtue of the additive material on the surface of the active particles. This release may be further improved as the carrier particles also have additive material applied to their surfaces. This application can be achieved by simple gentle blending or co-milling, for example as described in WO 97/03649.

However, the combination of large carrier particles and fine active particles has its disadvantages. It can only be effectively used with a relatively low (usually only up to 5%) drug content. As greater proportions of fine particles are used, more and more of the fine particles fail to become attached to the large carrier particles and segregation of the powder formulation becomes a problem. This, in turn, can lead to unpredictable and inconsistent dosing. The powder also becomes more cohesive and difficult to handle.

Furthermore, the size of the carrier particles used in a dry powder formulation can be influential on segregation.

Segregation can be a catastrophic problem in powder handling during manufacture and the filling of devices or device components (such as capsules or blisters) from which the powder is to be dispensed. Segregation tends to occur where ordered mixes cannot be made sufficiently stable. Ordered mixes occur where there is a significant disparity in powder particle size. Ordered mixes become unstable and prone to segregation when the relative level of the fine component increases beyond the quantity which can adhere to the larger component surface, and so becomes loose and tends to separate from the main blend. When this happens, the instability is actually exacerbated by the addition of anti-adherents/glidants such as FCAs.

In the case of dry powder formulations of micron-sized drug, and typical 60 to 150µm sized carrier, this instability tends to occur once drug content exceeds a few percent, the exact amount is dependant on the drug. However, it has been found that a carrier with a particle size of <30µm tends not to exhibit this instability. This is thought to be due to the fine carrier particles having relatively higher surface area compared to the coarse carrier particles, and the similarity between the size of the active particles and the carrier particles. Such fine carrier particles are not often used, mainly because of their poor flow characteristics, as discussed above.

According to another embodiment of the present disclosure, the 3-component system comprises the composite active particles made according to the present disclosure, together with fine excipient particles. Such excipient particles have a particle size of 30µm or less, preferably 20µm or less and more preferably 10µm or less. The excipient particles advantageously have a particle size of 30 to 5µm.

One would expect such a powder formulation, made up of only fine particles with a particle size of less than 10µm, to suffer from the cohesion and flowability problems observed with formulations comprising just fine active particles. The active particles do not coat the fine excipient particles, as they do the large carrier particles, because of the different forces existing between fine particles and fine and large particles.

However, where the powder formulation comprises composite active particles according to the present disclosure and fine excipient particles, it has been surprisingly found that such formulations are efficiently dispensed by an active device. It has been found that the potentially poor flow characteristics or handleability of powders comprising only particles with a size of less than 10µm are not significant when the powder is dispensed using an active inhaler device.

As mentioned above, the active device causes turbulence within the blister, capsule or other powder storage means. This means that even powders with fine excipient particles can be extracted. Furthermore, the presence of the composite active particles means that the agglomerates formed from the fine particles are not so stable that they are not broken up upon actuation of the inhaler device. Thus, it has been surprisingly found that compositions comprising the composite active particles of the present disclosure and fine particles of an inert excipient material, such as lactose, can be efficiently dispensed using an active inhaler device.

In another embodiment of the present disclosure, the fine excipient particles added to the composite active particles are themselves co-jet milled with additive material. The co-jet milling of the active particles with additive material and of the excipient particles with additive material can occur separately or together.

Co-jet milling the fine excipient particles with the additive material results in coating of the additive material on the surfaces of the excipient particles. This coating can further reduce the cohesiveness of the 3-component system and can further enhance deagglomeration upon actuation of the inhaler device.

Generally, flow of compositions comprising fine carrier particles is poor unless they are pelletised (e.g. as is done in the AstraZeneca product OXIS (registered trade mark). However, using the processes of the present invention, fine lactoses (e.g. Sorbolac 400 with a particle size of 1 to 15µm) have been produced which flow sufficiently well for use in DPIs with >5% drug, and up to approximately 30% and possibly 50% cohesive micronised drug. It should be noted that these beneficial properties are achieved without the need to resort to pelletisation, which has its own disadvantages of being difficult to do and generally decreasing FPFs.

Thus, the co-milling of the fine excipient particles and additive material in accordance with the present invention allows one to produce blends of active and excipient materials with a much greater range of active agent content than is possible using conventional carrier particles (i.e. >5%). The resultant dry powder formulations also benefit from improved aerosolisation.

In the present invention, different grinding and injection pressures may be used in order to produce particles with different coating characteristics. The invention also includes embodiments where different grinding and injection pressures are combined, to produce composite particles with desired properties, that is, to engineer the particles.

Co-jet milling may be carried out at grinding pressures between 0.1 and 12 bar. Varying the pressure allows one to control the degree of particle size reduction. At pressures in the region of 0.1-3 bar, and preferably 1-2 bar, the co-jet milling will primarily result in blending of the active and additive particles, so that the additive material coats the active particles. On the other hand, at 3-12 bar, and preferably 5-12 bar, the co-jet milling will additionally lead to particle size reduction.

In one embodiment, the jet milling is carried out at a grinding pressure of between 0.1 and 3 bar, to achieve blending of the active and additive particles. As discussed below in greater detail, when the co-jet milling of the present invention is carried out at such relatively low pressures, the resultant particles have been shown to perform well when dispensed using passive devices. It is speculated that this is because the particles are larger than those produced by co-jet milling at higher pressures and these relatively larger particles are more easily extracted from the blister, capsule or other storage means in the passive device, due to less cohesion and better flowability. Whilst such relatively large particles are easily extracted from the blister or capsule in an active device, they may result in throat deposition.

In another embodiment, the jet milling is carried out at a grinding pressure of between 3 and 12 bar, to achieve a reduction of the sizes of the active and additive particles. The co-jet milling at these relatively high pressures can produce extremely small composite active particles having a MMAD of between 3 and 0.5µm. These fine particle sizes are excellent for deep lung deposition, but they really need to be dispensed using an active inhaler device, as the powder formulations comprising such fine particles are actually rather "sticky". As discussed below, this stickiness does not pose a problem for active devices and is actually thought to be advantageous as it can slow the extraction of the powder so that the composite active particles travel more slowly in the powder plume generated by the device, thereby reducing throat deposition.

Tests were carried out whereby pre-micronised lactose (as a drug model) was co-jet milled in an MC50 Hosakawa Micron with 5% magnesium stearate. At 2 bar milling pressure, the resultant material had a d50 of approximately 3µm, whilst milling the same mixture at around 7 bar resulted in material with a d50 of about 1µm. Thus, when operating with a jet milling pressure of 0.1-3 bar little milling, that it is particle size reduction, is seen. From 3-12 bar milling pressure, increasing milling is seen, with the particle size reduction increasing with the increasing pressure. This means that the milling pressure may be selected according to the desired particle size in the resultant mixture.

As indicated above, co-jet milling at lower pressures produces powders which perform well in passive devices whilst powders milled at higher pressures perform better in active devices, such as Aspirair (trade mark).

The co-jet milling processes according to the present invention can also be carried out in two or more stages, to combine the beneficial effects of the milling at different pressures and/or different types of milling or blending processes. The use of multiple steps allows one to tailor the properties of the co-jet milled particles to suit a particular inhaler device, a particular drug and/or to target particular parts of the lung.

In one embodiment, the milling process is a two-step process comprising first jet-milling the drug on its own at high grinding pressure to obtain the very small particle sizes possible using this type of milling. Next, the milled drug is co-jet milled with an additive material. Preferably, this second step is carried out at a lower grinding pressure, so that the effect is the coating of the small active particles with the additive material.

This two-step process produces better results than simply co-jet milling the active material and additive material at a high grinding pressure. Experimental results discussed below show that the two-step process results in smaller particles and less throat deposition than simple co-jet milling of the materials at a high grinding pressure.

In another embodiment of the present invention, the particles produced using the two-step process discussed above subsequently undergo Mechano-Fusion. This final Mechano-Fusion step is thought to "polish" the composite active particles, further rubbing the additive material into the particles. This allows one to enjoy the beneficial properties afforded to particles by Mechano-Fusion, in combination with the very small particles sizes made possible by the co-jet milling.

The reduction in particle size may be increased by carrying out the co-jet milling at lower temperatures. Whilst the co-jet milling process may be carried out at temperatures between -20°C and 40°C, the particles will tend to be more brittle at lower temperatures, and they therefore fracture more readily so that the milled particles tend to be even smaller. Therefore, in another embodiment, the jet milling is carried out at temperatures below room temperature, preferably at a temperature below 10°C, more preferably at a temperature below 0°C.

Preferably, all of the particles are of a similar size distribution. That is, substantially all of the particles are within the size range of about 0 to about 50µm, of about 0 to about 20µm, of about 0 to 10µm, of about 0 to 5µm or of about 0 to 2µm.

In accordance with a second aspect of the present disclosure, a pharmaceutical dry powder composition for pulmonary inhalation is provided, comprising composite active particles made by a method according to the first aspect of the disclosure.

The MMAD of the composite active particles is preferably not more than 10µm, and advantageously it is not more than 5µm, more preferably not more than 3µm, even more preferably not more than 2µm, more preferably not more than 1.5µm, even more preferably not more than 1.2 µm and most preferably not more than 1µm.

Accordingly, advantageously at least 90% by weight of the composite active particles have a diameter of not more than 10µm, advantageously not more than 5µm, preferably not more than 3µm, even more preferably not more than 2.5µm, even more preferably not more than 2µm and more preferably not more than 1µm.

In a preferred embodiment of the present disclosure the resultant dry powder formulation has a reproducible FPF(ED) of at least 70%. Preferably, the FPF(ED) will be at least 80%, more preferably the FPF(ED) will be at least 85%, and most preferably the FPF(ED) will be at least 90%.

In a further preferred embodiment, the dry powder formulation has a reproducible FPF(MD) of at least 60%. Preferably, the FPF(MD) will be at least 70%, more preferably the FPF(MD) will be at least 80%, and most preferably the FPF(MD) will be at least 85%.

As illustrated by the experimental results set out below, it has been surprisingly found that co-milling active particles with additive particles using jet milling results in composite active particles having significantly better FPF and FPD than those produced by co-milling using Mechano-Fusion, when the powders are dispensed using the active inhaler device Aspirair (trade mark).

This unexpected improvement in the FPF and FPD of the powder formulations prepared is thought to be due to the following factors. Firstly, the milling process results in very small particles. Secondly, there appears to be only partial coverage of the particles with the force control agent and this means that some of the particle cohesion is retained, affording better powder handleability despite the very small particle sizes.

Co-jet milling has surprisingly been found to be capable of significantly reducing the median primary particle size of active particles (for example, from 3 or 2µm to 1µm), while also allowing good aerosolisation from a delivery device. This further reduction in primary particle size is considered to be advantageous for delivery of systemically targeted molecules to the deep lung. The benefit here is to co-jet mill active particles with additive particles in order to reduce primary particle size while still achieving a reduction in the level of powder cohesion and adhesion by coating the particles for additive material.

### Test Methods

All materials were evaluated in the Next Generation Impactor (NGI). Details of the test are provided in each case.

Formulations were processed using:
1) The Hosokawa Micron Mechano-Fusion AMS Mini system. This system was operated with a novel rotor, providing a 1mm compression gap; and
2) The Hosokawa Micron AS50 spiral jet mill.

The in-vitro testing was performed using an Aspirair (trade mark) device, which is an active inhaler device.

The formulations were composed of one or more of the following constituents:
Magnesium stearate (standard grade)
L-Leucine (Ajinomoto) and jet milled by Micron Technologies
Sorbolac 400 lactose
Micronised clobozam
Micronised apomorphine hydrochloride
Micronised lactose
Re-condensed Leucine (Aerocine)

### Comparison of Co-Jet Milled and Mechano-Fused Formulations (clobozam)

The following is a comparison of 2-component systems comprising co-jet milled or Mechano-Fused active particles and additive material.

1.01g of micronised clobozam was weighed out, and then gently pressed through a 300µm metal sieve, using the rounded face of a metal spatula. This formulation was recorded as "3A".

9.37g of micronised clobozam was then combined with 0.50g of micronised L-leucine in the Mechano-Fusion system. The material was processed at a setting of 20% power for 5 minutes, followed by a setting of 80% power for 10 minutes. This material was recorded as "4A". After blending, this powder was then gently pushed through a 300µm metal sieve with a spatula. This material was recorded as "4B".

9.57g of micronised clobozam was then combined with 0.50g of magnesium stearate in the Mechano-Fusion system. The material was processed at a setting of 20% power for 5 minutes, followed by a setting of 80% power for 10 minutes. This material was recorded as "5A". After blending, this powder was rested overnight, and then was gently pushed through a 300µm metal sieve with a spatula. This material was recorded as "5B".

9.5g of micronised clobozam was then combined with 0.50g of micronised L-leucine in the Mechano-Fusion system. The material was processed at a relatively low speed setting of 20% power for 5 minutes. This process was intended only to produce a good mix of the components. This material was recorded as "6A".

6.09g of "6A" fed at approximately 1g per minute into an AS50 spiral jet mill, set with an injector pressure of about 7 bar and a grinding pressure of about 5 bar. The resulting material was recovered and recorded as "6B".

After milling, this powder was rested overnight, and then was gently pushed through a 300µm metal sieve with a spatula. This material was recorded as "6C".

9.5g of micronised clobozam was then combined with 0.50g of magnesium stearate in the Mechano-Fusion system. The material was processed at a setting of 20% power for 5 minutes. This material was recorded as "7A".

6.00g of "7A" was fed at approximately 1g per minute into the AS50 spiral jet mill, set with an injector pressure of about 7 bar and a grinding pressure of about 5 bar. The resulting material was recovered and recorded as "7B".

After milling, this powder was gently pushed through a 300µm metal sieve with a spatula. This material was recorded as "7C".

A batch of re-condensed leucine (also referred to as "Aerocine") was produced by subliming to vapour a sample of leucine in a tube furnace, and re-condensing as a very finely dispersed powder as the vapour cooled. This batch was identified as "8A".

9.5g of micronised clobozam was then combined with 0.50g of Aerocine, in the Mechano-Fusion system. The material was processed at a setting of 20% power for 5 minutes, followed by a setting of 80% power for 10 minutes. This material was recorded as "8B". After blending, this powder was rested overnight, and then was gently pushed through a 300µm metal sieve with a spatula. This material was recorded as "8C".

9.5g of micronised clobozam was combined with 0.50g of Aerocine in the Mechano-Fusion system. The material was processed at a setting of 20% power for 5 minutes. 7.00g of this powder was then fed into the AS50 spiral jet mill, set with an injector pressure of about 7 bar and a grinding pressure of about 5 bar. The resulting material was recovered and recorded as "9A".

After milling, this powder was gently pushed through a 300µm metal sieve with a spatula. This material was recorded as "9B".

A number of foil blisters were filled with approximately 2mg of the following clobozam formulations:
3A - no milling & no additive material
4B - leucine & Mechano-Fused
5B - magnesium stearate & Mechano-Fused
6C - leucine & co-jet milled
7C - magnesium stearate & co-jet milled
8C - Aerocine & co-jet milled
9B - Aerocine & Mechano-Fused.

These formulations were then fired from an Aspirair device into an NGI at a flow rate of 60l/m. The Aspirair was operated under 2 conditions for each formulation: with a reservoir of 15ml of air at 1.5 bar or with a reservoir of 30ml of air at 0.5 bar.

Full details of the results are attached. The impactor test results are summarised in Tables 29, 30 and 31 below.

**Table 29**

| **Formulation** | **MD (mg)** | **DD (mg)** | **FPD (mg) (<5µm)** | **MMAD** |
|---|---|---|---|---|
| **3A** | 2.04 | 1.12 | 0.88 | 2.91 |
| 0.5 bar 30ml | | | | |
| **3A** | 1.92 | 1.74 | 1.23 | 2.86 |
| 1.5 bar 15ml | | | | |
| **4B** | 1.84 | 1.48 | 0.82 | 3.84 |
| 0.5 bar 30ml | | | | |
| **4B** | 1.80 | 1.56 | 0.81 | 3.32 |
| 1.5 bar 15ml | | | | |
| **5B** | 1.84 | 1.53 | 1.17 | 2.34 |
| 0.5 bar 30ml | | | | |
| **5B** | 1.85 | 1.55 | 1.12 | 2.22 |
| 1.5 bar 15ml | | | | |
| **6C** | 1.93 | 1.80 | 1.67 | 2.11 |
| 0.5 bar 30ml | 1.86 | 1.73 | 1.62 | 2.11 |
| **6C** | 1.97 | 1.86 | 1.67 | 2.07 |
| 1.5 bar 15ml | | | | |
| **6C** | 1.74 | 1.65 | 1.46 | 2.03 |
| 1.5 bar 15ml | | | | |
| (silicon coated plates) | | | | |
| **7C** | 2.06 | 1.99 | 1.87 | 1.97 |
| 0.5 bar 30ml | | | | |
| **7C** | 1.89 | 1.78 | 1.63 | 1.79 |
| 1.5 bar 15ml | | | | |
| **8C** | 1.82 | 1.73 | 1.62 | 2.02 |
| 0.5 bar 30ml | | | | |
| **8C** | 1.81 | 1.74 | 1.57 | 2.01 |
| 1.5 bar 15ml | | | | |
| **9B** | 1.88 | 1.73 | 1.04 | 3.48 |
| 0.5 bar 30ml | | | | |
| **9B** | 1.80 | 1.64 | 0.94 | 3.12 |
| 1.5 bar 15ml | | | | |

**Table 30**

| **Formulation** | **FPF(MD) % (<5µm)** | **FPF(ED) % (<5µm)** | **FPF(ED) % (<3µm)** | **FPF(ED) % (<2µm)** | **FPF(ED) % (<1µm)** |
|---|---|---|---|---|---|
| **3A** | 43 | 78 | 49 | 32 | 17 |
| 0.5 bar 30ml | | | | | |
| **3A** | 64 | 71 | 45 | 24 | 6 |
| 1.5 bar 15ml | | | | | |
| **4B** | 45 | 55 | 28 | 15 | 7 |
| 0.5 bar 30ml | | | | | |
| **4B** | 45 | 52 | 30 | 18 | 9 |
| 1.5 bar 15ml | | | | | |
| **5B** | 64 | 77 | 54 | 42 | 30 |
| 0.5bar 30ml | | | | | |
| **5B** | 61 | 72 | 52 | 38 | 25 |
| 1.5 bar 15ml | | | | | |
| **6C** | 87 | 93 | 77 | 44 | 8 |
| 0.5 bar 30ml | 87 | 94 | 76 | 44 | 9 |
| **6C** | 85 | 90 | 73 | 44 | 10 |
| 1.5 bar 15ml | | | | | |
| **6C** | 84 | 89 | 74 | 45 | 8 |
| 1.5 bar 15ml | | | | | |
| (silicon coated plates) | | | | | |
| **7C** | 91 | 94 | 79 | 50 | 14 |
| 0.5 bar 30ml | | | | | |
| **7C** | 86 | 92 | 82 | 56 | 16 |
| 1.5 bar 15ml | | | | | |
| **8C** | 89 | 93 | 79 | 48 | 12 |
| 0.5 bar 30ml | | | | | |
| **8C** | 87 | 90 | 76 | 46 | 9 |
| 1.5 bar 15ml | | | | | |
| **9B** | 55 | 60 | 34 | 24 | 15 |
| 0.5 bar 30ml | | | | | |
| **9B** | 52 | 57 | 34 | 24 | 15 |
| 1.5 bar 15ml | | | | | |

**Table 31**

| **Formulation** | ***recovery** | ***throat** | ***blister** | ***device** |
|---|---|---|---|---|
| **3A** | 102% | 3% | 1% | 43% |
| 0.5 bar 30ml | | | | |
| **3A** | 96% | 15% | 1% | 8% |
| 1.5 bar 15ml | | | | |
| **4B** | 97% | 15% | 7% | 12% |
| 0.5 bar 30ml | | | | |
| **4B** | 95% | 27% | 6% | 8% |
| 1.5 bar 15ml | | | | |
| **5B** | 97% | 7% | 13% | 4% |
| 0.5 bar 30ml | | | | |
| **5B** | 98% | 14% | 12% | 4% |
| 1.5 bar 15ml | | | | |
| **6C** | 97% | 2% | 1% | 6% |
| 0.5 bar 30ml | 101% | 3% | 1% | 5% |
| **6C** | 104% | 6% | 3% | 3% |
| 1.5 bar 15ml | | | | |
| **6C** | 91% | 8% | 1% | 4% |
| 1.5 bar 15ml | | | | |
| (silicon coated plates) | | | | |
| **7C** | 110% | 2% | 1% | 3% |
| 0.5 bar 30ml | | | | |
| **7C** | 99% | 6% | 2% | 3% |
| 1.5 bar 15ml | | | | |
| **8C** | 99% | 3% | 1% | 4% |
| 0.5 bar 30ml | | | | |
| **8C** | 95% | 6% | 1% | 3% |
| 1.5 bar 15ml | | | | |
| **9B** | 96% | 16% | 2% | 7% |
| 0.5 bar 30ml | | | | |
| **9B** | 95% | 26% | 4% | 5% |
| 1.5 bar 15ml | | | | |

From these results it can be seen that the co-jet milled formulations exhibited exceptional FPFs when dispensed from an active dry powder inhaler device. The FPFs observed were significantly better that those of the Mechano-Fused formulations and those formulations which did not include an additive material. This improvement would appear to be largely due to reduced throat deposition, which was less than 8% for the co-jet milled formulations, compared to 15% for the pure drug and up to 27% for the Mechano-Fused formulations.

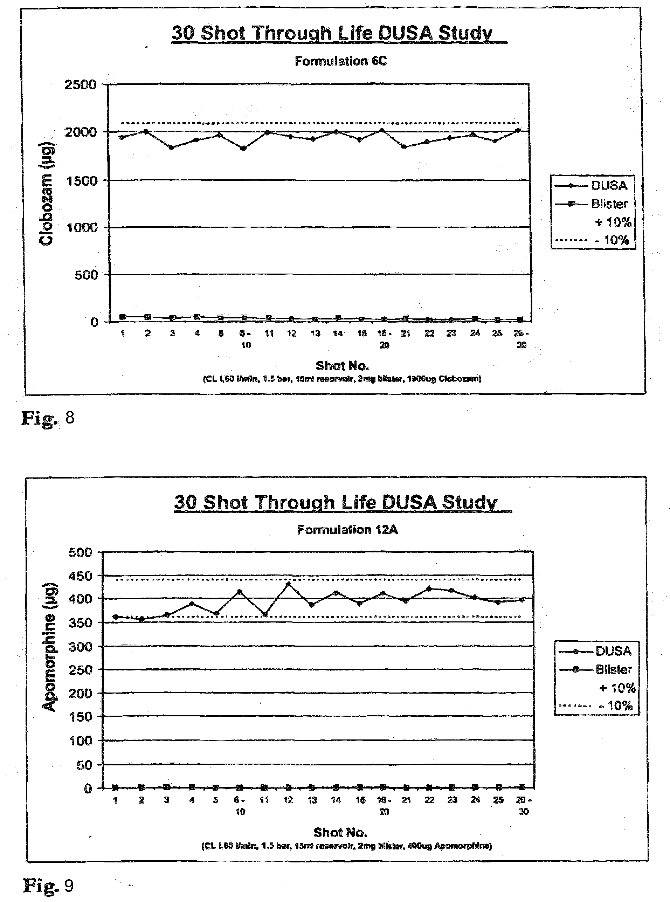

The reproducibility of the FPFs obtained was also tested. Through life dose uniformity for the primary candidate, 6C, the preparation of which is described above, was tested by firing 30 doses, with the emitted doses collected by DUSA. Through life dose uniformity results are presented in the graph of Figure 8.

The mean ED was 1965µg, with an RSD (relative standard deviation) of 2.8%. This material consequently demonstrated excellent through life dose reproducibility.

The results of particle size testing by Malvern of these powdered materials are provided in the following figures. The particle size distributions indicate the level of size reduction obtained by the co-milling.

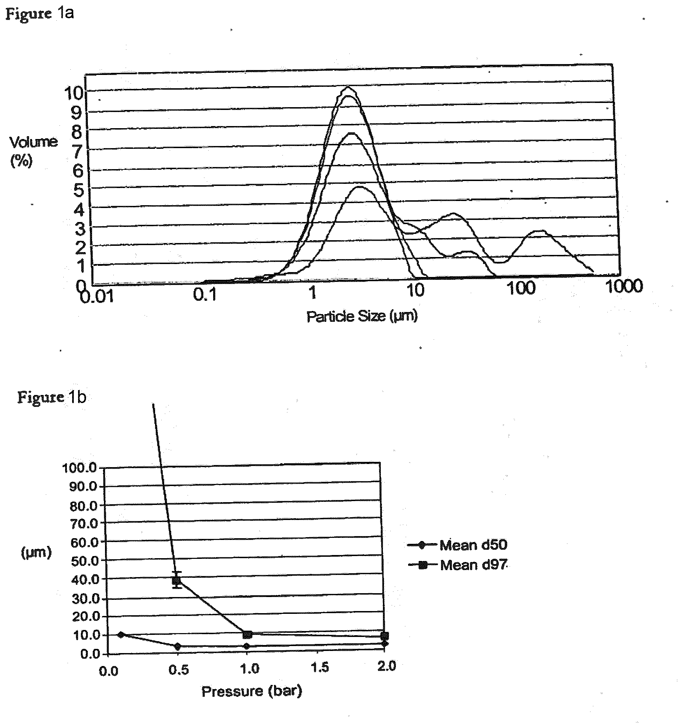

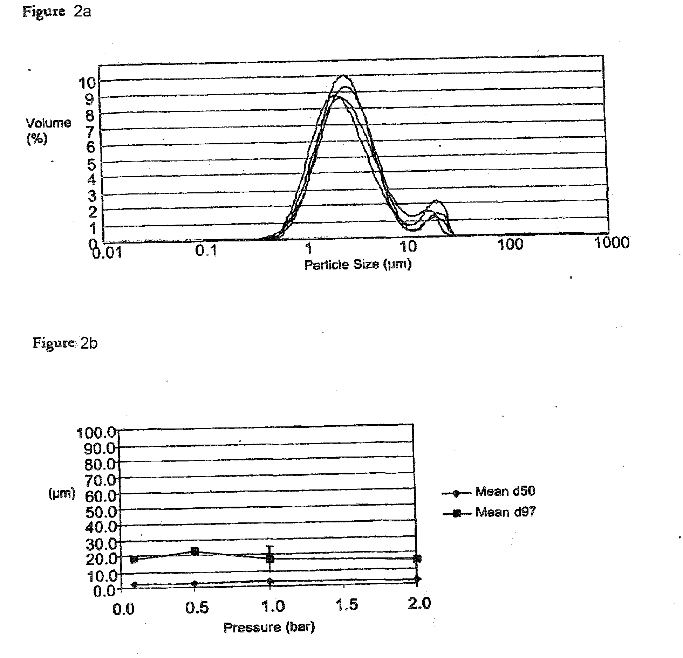

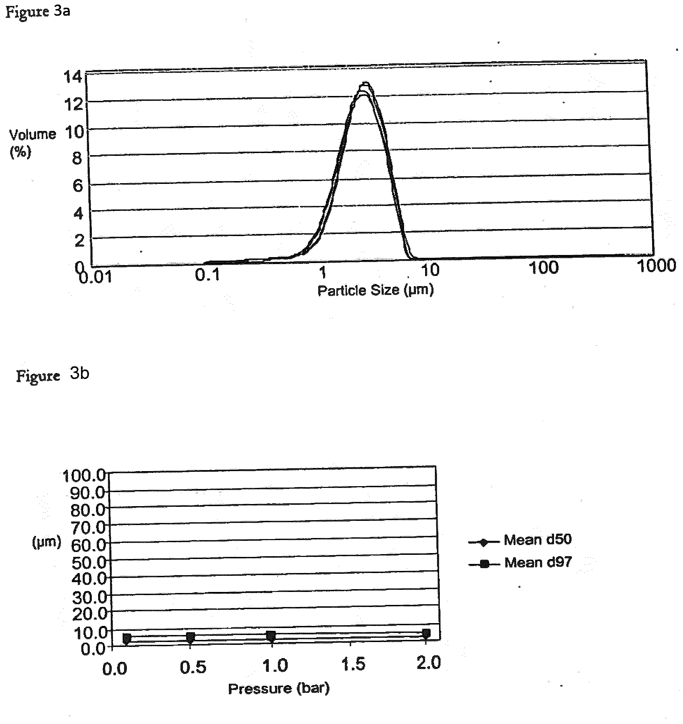

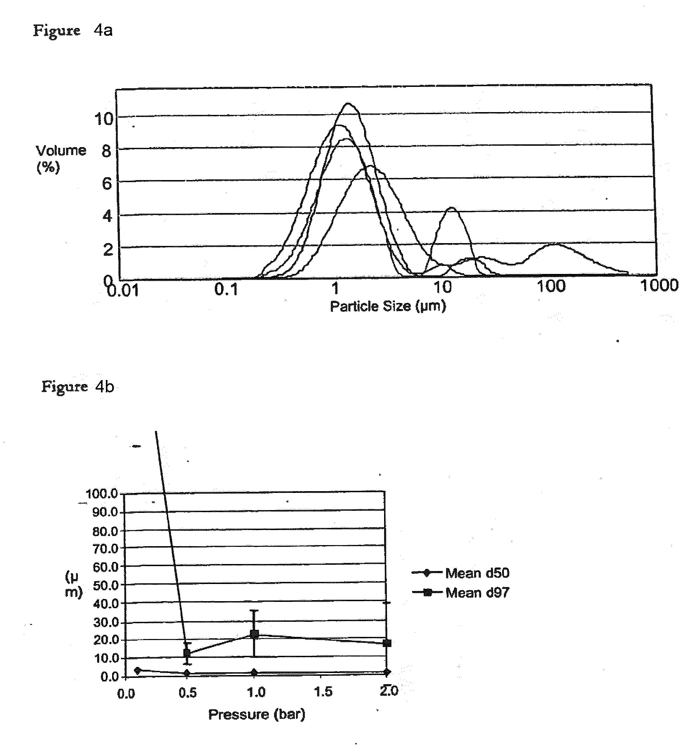

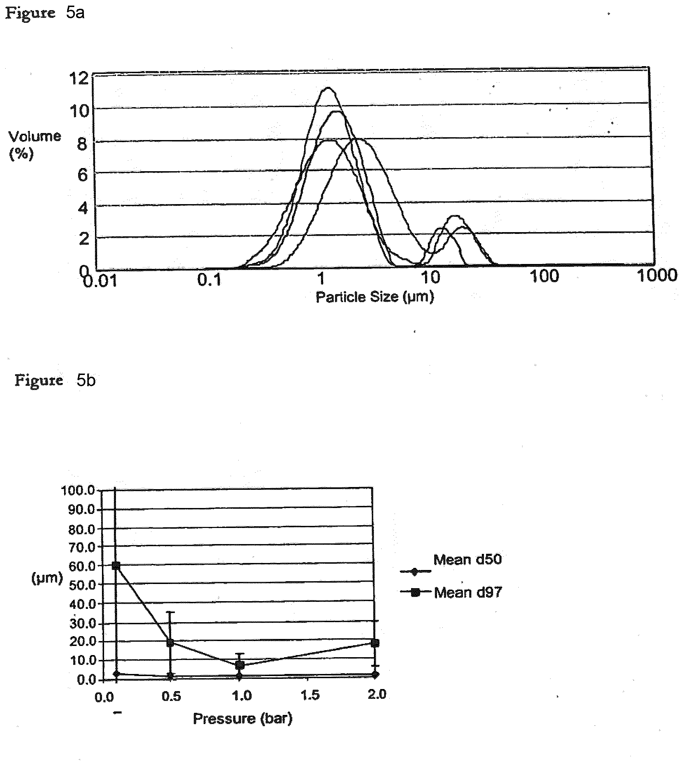

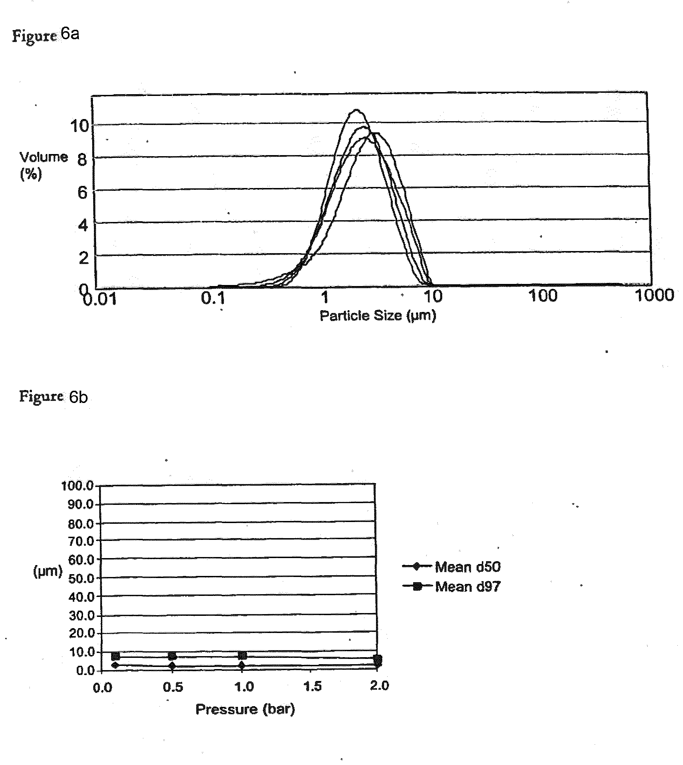

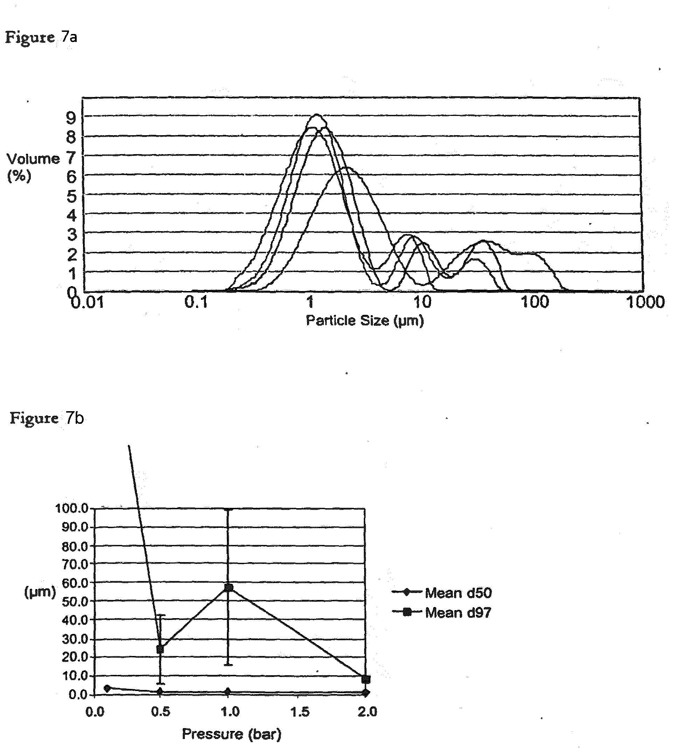

The results of dispersion testing of these powdered materials are provided in the Figures 1A to 7B. The particle size distributions indicate both the level of size reduction obtained by the co-milling, and the level of dispersion efficiency at varied pressures. The d50 and d97 plots provide a further indication of this dispersibility of the powders as a function of pressure.

The graphs in Figures 1A to 7A figures show the particle size distribution, with the four curves representing powder jet-milled at different pressures, namely at 2.0 bar, 1.0 bar, 0.5 bar and at 0.1 bar. The graphs in Figure 1B to 7B show the level of dispersion efficiency at different pressures, in terms of d50 and d97.
Figures 1A and 1B are the results of testing formulation "3A";
Figures 2A and 2B are the results of testing formulation "4B";
Figures 3A and 3B are the results of testing formulation "5B";
Figures 4A and 4B are the results of testing formulation "6C";
Figures 5A and 5B are the results of testing formulation "7C";
Figures 6A and 6B are the results of testing formulation "8C"; and
Figures 7A and 7B are the results of testing formulation "9B".

From the graphs, one can see that formulation 5B exhibited much the best dispersion.

This set of dispersibility tests shows that the MechanoFused powders disperse more easily at lower pressures than the original drug, and that magnesium stearate gives the best dispersion within these, followed by Aerocine and leucine. The co-jet milled powders do not appear to disperse any more easily in this test than the original drug, however the primary particle sizes (d50) are reduced.

### Comparison of Co-Jet Milled and Mechano-Fused Formulations (Apomorphine)

In order to establish the effect of co-jet milling on different active agents, apomorphine hydrochloride formulations with fine carrier particles (i.e. a 3-component system) were prepared and tested.

19.0g of Sorbolac 400 lactose and 1.0g of micronised L-leucine were combined in the Mechano-Fusion system. The material was processed at a setting of 20% power for 5 minutes, followed by a setting of 80% power for 10 minutes. This material was recovered and recorded as "2A".

15.0g of apomorphine hydrochloride and 0.75g of micronised L-leucine were combined in the Mechano-Fusion system. The material was processed at a setting of 20% power for 5 minutes, followed by a setting of 80% power for 10 minutes. This material was recovered and recorded as "2B".

2.1g "2B" plus 0.4g micronised leucine were blended by hand in a mortar and pestle for 2 minutes. 2.5g micronised lactose was added and blended for a further 2 minutes. 5g micronised lactose was added and blended for another 2 minutes. This mixture was then processed in the AS50 Spiral jet mill using an inlet pressure of 7 bar and a grinding pressure of 5 bar, feed rate 5ml/min. This powder was gently pushed through a 300µm metal sieve with a spatula. This material was recorded as "10A".

1.5g "10A" was combined with 0.20g micronised L-leucine and 3.75g of Sorbolac 400 lactose by hand in a mortar with a spatula for 10 minutes. This powder was gently pushed through a 300µm metal sieve with a spatula. This material was recorded as "10B".

9g micronised apomorphine HCl plus 1g micronised leucine were placed in the Mechano-Fusion system and processed at 20% (1000rpm) for 5 minutes. This initial blend was then processed in the AS50 Spiral jet mill using an inlet pressure of 7 bar and a grinding pressure of 5 bar, feed rate 5ml/min. This material was recorded as "11A".

After blending, this powder was rested overnight, and then was gently passed through a 300µm metal sieve by shaking. This material was recorded as "11B".

2g micronised apomorphine HCl plus 0.5g micronised leucine were blended by hand in mortar and pestle for 2 minutes. 2.5g micronised lactose was added and blended for a further 2 minutes. Then 5g micronised lactose was added and blended for another 2 minutes. This mixture was then processed in the AS50 Spiral jet mill using an inlet pressure of 7 bar and a grinding pressure of 5 bar, feed rate 5ml/min. This powder was gently pushed through a 300µm metal sieve with a spatula. This material was recorded as "12A".

16.5g of Sorbolac 400 and 0.85g of micronised leucine were placed in the Mechano-Fusion system and processed at 20% (1000rpm) for 5 minutes then at 80% (4000rpm) for 10 minutes. This material was recorded as "13A".

0.5g micronised apomorphine HCl plus 2.0g "13A" were blended by hand in a mortar with a spatula for 10 minutes. This powder was gently pushed through a 300µm metal sieve with a spatula. This material was recorded as "13B".

A number of foil blisters were filled with approximately 2mg of the following formulations:
10A - 20% apomorphine HCl, 5% 1-leucine, 75% micronised lactose (co-jet milled)
10C - 26.2% apomorphine HCl, 5% 1-leucine, 68.7% sorbolac (geometric)
11B - 95% apomorphine HCl, 5% 1-leucine (co-jet milled)
12A - 20% apomorphine HCl, 5% leucine, 75% micronised lactose (all co-jet milled)
13B - 20% apomorphine HCl, 5% 1-leucine, 75% Sorbolac 400 (leucine & Sorbolac Mechano-Fused)

These were then fired from an Aspirair device into an NGI at a flow rate of 60l/m. The Aspirair was operated with a reservoir of 15ml at 1.5 bar. Each in vitro test was conducted once to screen, and then the selected candidates were repeated. Further candidates were also repeated in ACI at 60 l/m.

**Table 32**

| **Formulation 2mg, 1.5 bar 15ml reservoir 60 l/min** | **MD (µg)** | **DD (µg)** | **FPD (<5µm) (µg)** | **MMAD** |
|---|---|---|---|---|
| **10A** | 384 | 356 | 329 | 1.78 |
| **13B** | 359 | 327 | 200 | 1.54 |
| | (1793) | (1635) | (1000) | |
| **10C** | 523 | 492 | 374 | 1.63 |
| **11B** | 1891 | 1680 | 1614 | 1.36 |
| | 1882 | 1622 | 1551 | 1.44 |
| | 1941 | 1669 | 1601 | 1.49 |
| | | | | |
| Ave. | 1905 | 1657 | 1589 | 1.43 |
| SD | 32 | 31 | 33 | 0.07 |
| RSD | 1.7 | 1.9 | 2.1 | 4.6 |
| **11B** | 1895 | 1559 | 1514 | 1.58 |
| | 1895 | 1549 | 1485 | 1.62 |
| | 1923 | 1565 | 1504 | 1.62 |
| ACI | | | | |
| Ave. | 1904 | 1558 | 1501 | 1.61 |
| SD | 16 | 8 | 15 | 0.02 |
| RSD | 1 | 1 | 1 | 1 |
| **12A** | 414 | 387 | 363 | 1.63 |
| | 410 | 387 | 363 | 1.66 |
| | 406 | 378 | 355 | 1.68 |
| | | | | |
| Ave. | 410 | 384 | 360 | 1.66 |
| SD | 4 | 5 | 5 | 0.03 |
| RSD | 1 | 1 | 1 | 2 |
| | | | | |
| Total ave. | 2050 | 1920 | 1800 | |
| **12A** | 395 | 365 | 341 | 1.80 |
| | 411 | 385 | 360 | 1.85 |
| | 400 | 370 | 349 | 1.84 |
| ACI | | | | |
| Ave. | 402 | 373 | 350 | 1.83 |
| SD | 8 | 10 | 10 | 0.04 |
| RSD | 2 | 3 | 3 | 2 |
| | | | | |
| Total ave. | 2011 | 1866 | 1750 | |

**Table 33**

| **Formulation 2mg, 1.5 bar 15ml reservoir 60 l/min** | **FPF(MD) % (<5um)** | **FPF(ED) % (<5um)** | **FPF(ED) % (<3um)** | **FPF(ED) % (<2um)** | **FPF(ED) % (<1um)** |
|---|---|---|---|---|---|
| **10A** | 86 | 93 | 87 | 60 | 13 |
| **13B** | 56 | 61 | 52 | 42 | 19 |
| **10C** | 72 | 76 | 67 | 51 | 16 |
| **11B** | 85 | 96 | 95 | 81 | 24 |
| | 82 | 96 | 93 | 77 | 22 |
| | 82 | 96 | 92 | 74 | 20 |
| | | | | | |
| Ave. | 83 | 96 | 93 | 77 | 22 |
| SD | | 0 | 1.5 | 3.5 | 2 |
| RSD | | 0 | 1.6 | 4.5 | 9.1 |
| **11B** | 80 | 97 | 94 | 74 | 14 |
| | 78 | 96 | 93 | 70 | 14 |
| | 78 | 96 | 94 | 72 | 12 |
| ACI | | | | | |
| Ave. | 79 | 96 | 94 | 72 | 13 |
| SD | | 1 | 1 | 2 | 1 |
| RSD | | 1 | 1 | 3 | 9 |
| **12A** | 88 | 94 | 89 | 68 | 13 |
| | 89 | 94 | 89 | 66 | 12 |
| | 87 | 94 | 88 | 64 | 12 |
| | | | | | |
| Ave. | 88 | 94 | 89 | 66 | 12 |
| SD | | 0 | 1 | 2 | 1 |
| RSD | | 0 | 1 | 3 | 5 |
| **12A** | 86 | 94 | 85 | 57 | 9 |
| | 88 | 93 | 84 | 55 | 8 |
| | 87 | 94 | 85 | 56 | 8 |
| ACI | | | | | |
| Ave. | 87 | 94 | 85 | 56 | 8 |
| SD | | 1 | 1 | 1 | 1 |
| RSD | | 1 | 1 | 2 | 7 |

**Table 34**

| **Formulation 2mg, 1.5 bar 15ml reservoir 60 l/min** | **Recovery** | **Throat** | **Blister** | **Device** |
|---|---|---|---|---|
| **10A** | 96% | 5% | 0.3% | 7% |
| **13B** | 94% | 29% | 3% | 6% |
| **10C** | 100% | 16% | 2% | 4% |
| **11B** | 101% | 2% | 0.6% | 10% |
| | 99% | 2% | 0.2% | 14% |
| | 102% | 2% | 0.3% | 14% |
| | | | | |
| Ave. | 101% | 2% | 0.4% | 13% |
| SD | 1.5 | 0 | 0.2 | 2.3 |
| RSD | 1.5 | 0 | 57 | 18 |
| **11B** | 100% | 1% | 0.5% | 17% |
| | 100% | 2% | 0.1% | 18% |
| | 101% | 2% | 0.4% | 18% |
| ACI | | | | |
| Ave. | 100% | 2% | 0.3% | 18% |
| SD | 1 | 1 | 0.2 | 1 |
| RSD | 1 | 35 | 62 | 3 |
| **12A** | 109% | 4% | 0.3% | 6% |
| | 108% | 4% | 0.2% | 6% |
| | 107% | 4% | 0.02% | 7% |
| | | | | |
| Ave. | 108 | 4% | 0.2 | 6% |
| SD | 1 | 0 | 0.1 | 1 |
| RSD | 1 | 0 | 82 | 9 |
| **12A** | 104% | 3% | 0.4% | 7% |
| | 108% | 4% | 0.2% | 6% |
| | 105% | 2% | 0.4% | 7% |
| ACI | | | | |
| Ave. | 106% | 3% | 0.3 | 7% |
| SD | 2 | 1 | 0.1 | 1 |
| RSD | 2 | 33 | 35 | 9 |

The co-jet milled formulations once again exhibited exceptional FPFs when it is dispensed using an active dry powder inhaler device. The improvement appears to be largely due to reduced throat deposition which was less than 5%, compared to between 16 and 29% for the Mechano-Fused formulations. "12A" was produced as a repeat of "10A", but excluding the Mechano-Fused pre-blend (to show it was not required).

The reproducibility of the FPFs obtained with the formulation 12A, the preparation of which is described above, was tested.

A number of foil blisters were filled with approximately 2mg of formulation 12A. Through life dose uniformity was tested by firing 30 doses, with the emitted doses collected by DUSA. Through life dose uniformity results are presented in the graph in Figure 9.

The mean ED was 389µg, with an RSD of 6.1% and the through life delivery of this drug-lactose formulation was very good.

In order to investigate the cause of the unexpected differences between the co-jet milled formulations and those prepared by Mechano-Fusion, formulations "11B", "10A" and "2C" were fired from an Aspirair and plume and vortex behaviour recorded on digital video. The images were studied in light of the above differences in throat deposition.

Video of plume behaviour indicated a difference between the co-jet milled formulations and Mechano-Fused formulations. Mechano-Fused formulations showed a highly concentrated fast moving bolus at the front of the air jet. Most powder appeared to have been emitted after approximately 40ms. Co-jet milled formulations showed a greater spread of the plume. The plume front moves at a similar velocity, but the front is less concentrated, appears to slow more quickly and powder is emitted for considerably longer (i.e. >200ms).

Video of the vortex showed that the Mechano-Fused powders enter the vortex within 10ms, whereas co-jet milled formulations take at least 30ms. Similarly the Mechano-Fused powders appeared quicker to leave the vortex, with the co-jet milled materials forming a more prolonged fogging of the vortex. The behaviour observed for co-jet milled materials was described as an increased tendency to stick, but then scour from the inside of the vortex.

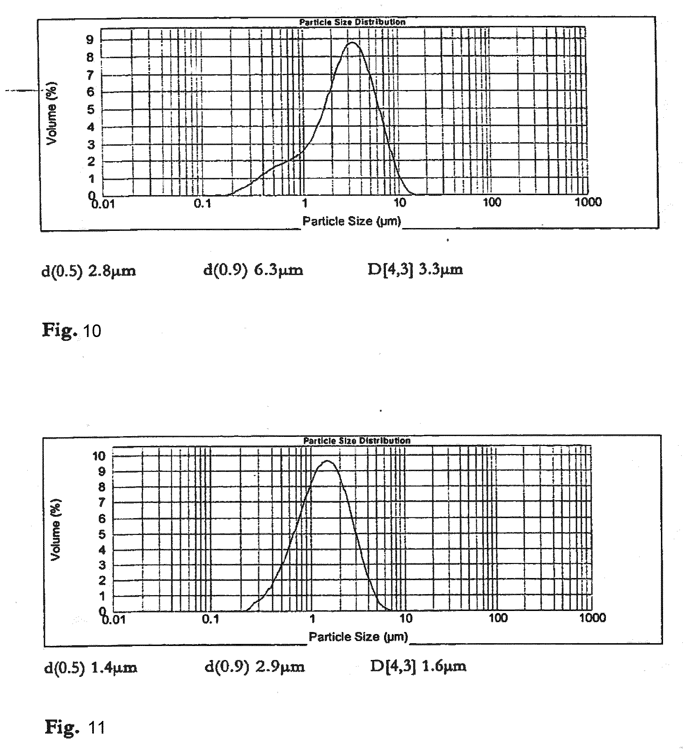

Particle size distributions of the raw materials and selected formulations were determined by Malvern particle sizer, via the Scirroco dry powder disperser. The data are summarised in the graphs shown in Figures 10 to 17.

Figure 10 shows the particle size distribution of the raw material micronised lactose (833704);

Figure 11 shows the particle size distribution of the raw material apomorphine;

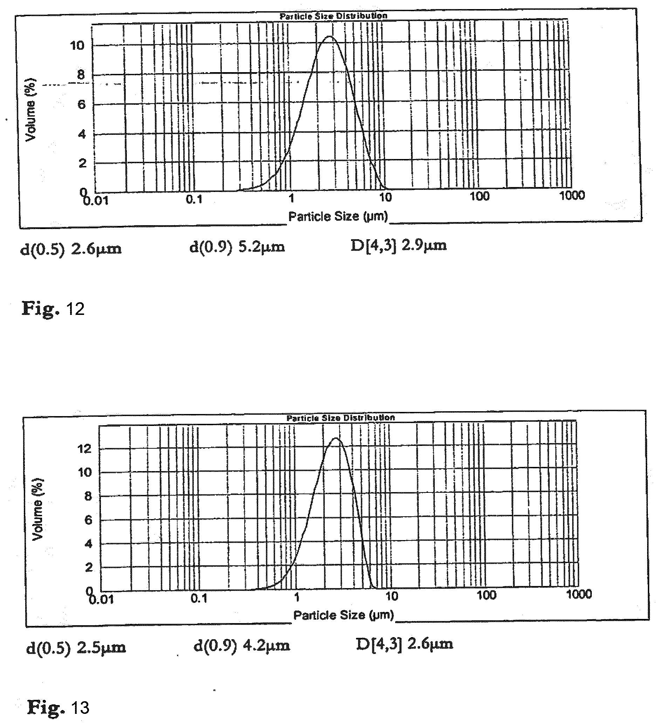

Figure 12 shows the particle size distribution of the raw material clobozam;

Figure 13 shows the particle size distribution of the clobozam formulation comprising 95% clobozam and 5% Mechano-Fused magnesium stearate;

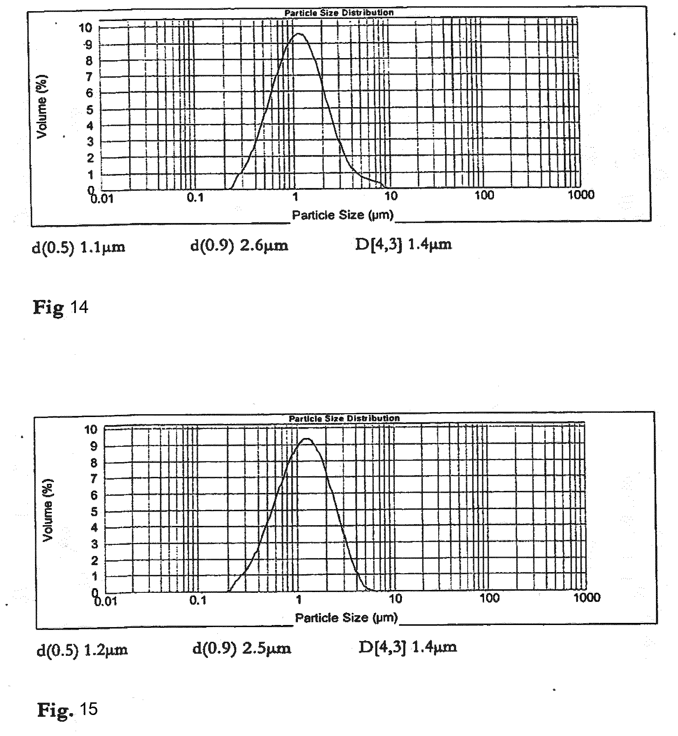

Figure 14 shows the particle size distribution of the clobozam formulation comprising 95% clobozam and 5% co-jet milled Aerocine;

Figure 15 shows the particle size distribution of the clobozam formulation comprising 95% clobozam and 5% co-jet milled leucine;

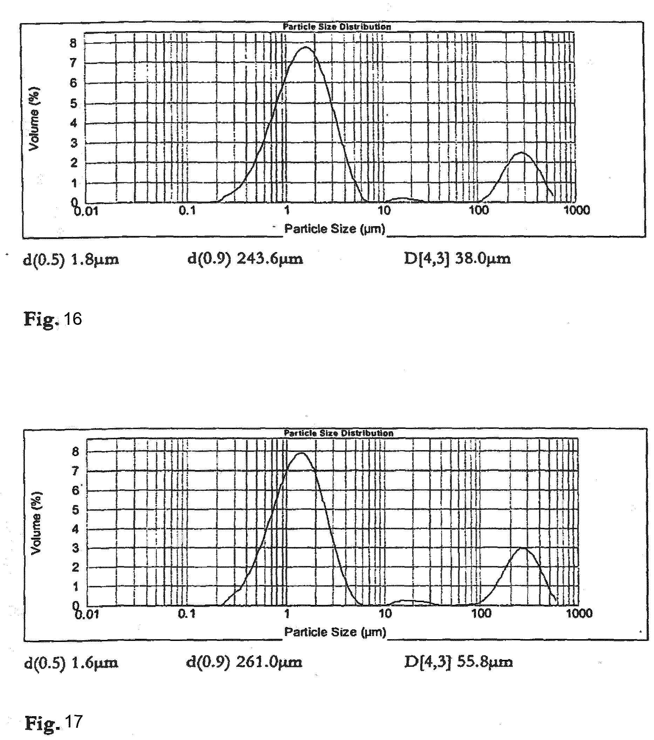

Figure 16 shows the particle size distribution of the apomorphine formulation comprising 75% lactose, 20% apomorphine and 5% co-jet milled leucine; and

Figure 17 also shows the particle size distribution of the apomorphine formulation comprising 75% lactose, 20% apomorphine and 5% co-jet milled leucine.

Where clobozam is co-jet milled with an additive material, a significant drop in particle size is observed. This is not seen for the clobozam Mechano-Fused formulation here.

With the apomorphine-lactose co-milled materials, the size distribution is low (d₅₀ 1.8 to 1.6), when compared to the particle size distribution of the micronised lactose which comprises 75% of the composition. However, size reduction is not detectable with respect to pure apomorphine, although it should be noted that this comprises only 20% of the powder composition.

In vitro data confirm that, surprisingly, Mechano-Fusion of active particles increased the throat deposition substantially. Mechano-Fusion has previously been associated with improvement in dispersibility from a passive device, and reduced throat deposition. In this case, Mechano-Fusion with magnesium stearate gives slightly lower throat deposition than Mechano-Fusion with leucine.

The throat deposition appears especially high for Mechano-Fused formulations containing leucine. It is speculated that this could be due to an agglomerating affect during Mechano-Fusion specific to leucine and not magnesium stearate, or an electrostatic effect specific to leucine.

However, surprisingly co-jet milling produces materials which, in comparison, give very low throat deposition, low device deposition and excellent dispersion from an active device. This co-jet milling also produces a significant further size reduction, for example, d50 changes from about 2.6µm to about 1µm for clobozam. When these factors are combined, a remarkable aerosolisation performance is obtained from the in-vitro tests. FPF(ED) are 90 to 96%. This excellent performance was obtained for leucine, Aerocine and magnesium stearate, and for 3 different formulations, including 2 different active agents, with or without lactose diluent.

The consequence of this is the achievement of a very low oropharangeal deposition to the patient, typically of approximately 5%. Given that both throat and upper airway deposition (corresponding to impactor throat and upper impactor stages) is reduced to a minimum, this will also result in a minimised tasteable component, and minimised fraction delivered to the GI tract. This corresponds to a 4-fold reduction in comparison to formulations without additive material.

It was noted that the co-jet milled materials were highly agglomerated in appearance, in contrast to the Mechano-Fused blends, which appeared as more free flowing powders.

Studies suggest that the difference between the performance of the co-jet milled and Mechano-Fused compositions is most apparent when the formulations are dispensed using an active device, such as Aspirair. Video of plume behaviour provided some indication of the reason for differences between the co-jet milled formulations and Mechano-Fused formulations. Mechano-Fused formulations showed a short fast bolus, whereas co-jet milled formulations showed a more drawn out plume. The "enhanced" flow properties of the Mechano-Fused powders appear to explain their worse Aspirair performance. A degree of powder hold-up within the device appears to be beneficial, allowing a less dense and extended plume to occur.

These video observations suggest the throat deposition difference is related to the powder lifetime within the vortex, with a longer lifetime giving reduced throat deposition. Lower aerosol concentration at the plume front, lower momentum of aerosol plume (with lower cloud density and smaller particle size) and greater opportunity to be de-agglomerated are possible contributors to this improvement. Also, there is also more material in the later, slower part of the plume. Furthermore, lower powder density in the cyclone appears to lead to better dispersion.

It is speculated that the fact that the powder formulations are difficult to extract from the blister actually enhances their delivery characteristics. It slows the extraction of the powder and so the active particles are travelling slower when they are expelled from the dispensing device. This means that the active particles do not travel at the front of the plume of powder created when the device is actuated and this means that the active particles are significantly less likely to impact on the throat of the user. Rather, the active particles are thought to be further back in the plume, which allows them to be inhaled and administered to the lung. Naturally, too much blister retention will be undesirable, as it will result in active agent remaining in the device after actuation.

In general, the co-milling of active particles with additive particles has yielded reduced device/blister retention compared to formulations prepared without additive particles. Mechano-Fusion was shown to give significantly greater blister retention than co-jet milling. The worst blister retention was seen for Mechano-Fused clobozam with magnesium stearate (13%). This appears related to the dusting nature of such formulations. The Mechano-Fused powders spread and flow more easily, which facilitates higher degrees of contact with the surfaces in bulk powder contact. The co-milled powders however are heavily agglomerated, so contact with surfaces is much reduced, and dust residues are also much less. The device retention also appears greater for Mechano-Fused than co-jet milled powders for clobozam. However, the device retention of apomorphine HCl co-jet milled with leucine appears notably high, at 13%. Device and blister retention does not appear substantially different between the 0.5 and 1.5 bar tests, except for the case of the unaltered pure clobozam, where device retention approaches 50% for the 0.5 bar test.

The tendency of a powder formulation to stick in the blister can be overcome in active devices, where a significant amount of turbulence is created within the blister when the device is actuated. However, this is not the case in a passive device. Therefore, the tendency of a formulation to stick in the blister will have a detrimental effect on the performance of a powder administered using a passive device. That said, as the active particles in the powder dispensed by a passive device are generally not moving as quickly as they would if dispensed by an active device, the problem of throat deposition (usually a result of the active particles travelling at the front of the powder plume) is not so great. Thus, it is clear that the properties of the active particles need to be tailored to the type of device used to dispense the powder.

Tests were carried out to compare the FPF achieved when the co-jet milled compositions are dispensed using passive and active devices. The experiments used a lactose model fired into a TSI. The results were as follows:

**Table 35**

| **Formulation** | **FPF(ED) %** | **FPF(MD) % (Cyclohaler)** | **FPF(MD) % (Aspirair)** |
|---|---|---|---|
| Micronised lactose | 32 | 18 | - |
| With 5% magnesium stearate (MgSt) in a conventional blender | 35 | 32 | 27 |
| 5% MgSt jet-milled at 2bar | 68 | 53 | 62 |
| 5% MgSt jet-milled at 7bar | 52 | 39 | 72 |
| 5% MgSt Mechano-Fused | 69 | 57 | 49 |

This shows that jet milled material which has been co-jet milled at low pressure is better in passive devices whilst high pressure jet milled materials perform better in active devices such as Aspirair.

### MechanoFused Budesonide with Magnesium Stearate

The magnesium stearate selected was a standard grade supplied by Avocado Research Chemicals Ltd., Lot H1028A. The drug used was budesonide.

This work was conducted using the Miat Monohaler. The work studied systems of magnesium stearate processed with budesonide. The blends were prepared by mechano-fusion using the Hosakawa AMS-MINI, blending for 60 minutes at approximately 4000 rpm.

Blends of budesonide and magnesium stearate were prepared at different weight percentages of magnesium stearate. Blends of 5% w/w and 10%w/w, were prepared and then tested. MSLIs and TSIs were carried out on the blends. The results, which are summarised below, indicate a high aerosolisation efficiency.

| **Formulation** | **FPF (ED)** | **FPD mg** | **ED mg** | **Method** |
|---|---|---|---|---|
| Budesonide:MgSt (5%w/w) | 73% | 1.32 | 1.84 | MSLI |
| Budesonide:MgSt (10%w/w) | 80% | 1.30 | 1.63 | TSI |

### Mechano-fused Budesonide with Fine Lactose and Magnesium Stearate

A further study was conducted to look at the Mechano-Fusion of a drug with both a force control agent and a fine lactose. The force control agent used was magnesium stearate (Avocado) and the fine lactose was Sorbolac 400 (Meggle). The drug used was budesonide (2M00M0-0019427). The blends were prepared by Mechano-Fusion using the Hosakawa AMS-MINI, blending for 60 minutes at approximately 4000 rpm.

Formulations were prepared using the following concentrations of budesonide, magnesium stearate and Sorbolac 400:
5% w/w budesonide, 6% w/w MgSt, 89% w/w Sorbolac 400,
20% w/w Budesonide, 6% w/w MgSt, 74% w/w Sorbolac 400

TSIs and MSLIs were performed on the blends. The results summarised below indicate that, as the amount of budesonide in the blends increased, the FPF results increased. Device and capsule retention were notably low in these dispersion tests (>5%)

| **Formulation** | **FPF(ED) (TSI)** | **FPF(ED) (MSLI)** |
|---|---|---|
| 5:6:89 | 66.0% | 70.1% |
| 20:6:74 | 75.8% | - |

As an extension to this work, different blending methods of Budesonide, MgSt and Sorbolac 400 were investigated further.

Two formulations were prepared in the Glen Creston Grindomix. This mixer is a conventional food-processor style bladed mixer, with 2 parallel blades.

The first of these formulations was a 5% w/w budesonide, 6%w/w MgSt, 89% w/w Sorbolac 400 blend prepared by mixing all components together at 2000rpm for 20 minutes. The formulation was tested by TSI and the results, when compared to those for the mechano-fused blends, showed the Grindomix blend to give lower FPF results (see table below).

The second formulation was a blend of 90% w/w of mechanofused magnesium stearate:Sorbolac 400 (5:95) pre-blend and 10% w/w budesonide blended in the Grindomix for 20 minutes. The formulation was tested by TSI and MSLI.

It was also observed that this formulation had notably good flow properties for a material comprising such fine particles: this was associated with the Mechano-fusion process.

| **Formulation** | **FPF (TSI)** | **FPF (MSLI)** |
|---|---|---|
| Grindomix 5:6:89% | 57.7 | - |
| Grindomix 10% budesonide (mechanofused pre-blend) | 65.9 | 69.1 |

### Mechano-Fused Salbutamol with Fine Lactose and Magnesium Stearate

A further study was conducted to look at the Mechano-Fusion of a further drug with both a force control agent and the fine lactose. The force control agent used was magnesium stearate and the fine lactose was Sorbolac 400 (Meggle). The drug used was micronised salbutamol sulphate. The blends were prepared by Mechano-Fusion using the Hosakawa AMS-MINI, blending for 10 minutes at approximately 4000 rpm.

Formulations prepared were:
20% w/w salbutamol, 5% w/w MgSt, 75% w/w Sorbolac 400
20% w/w salbutamol, 2% w/w MgSt, 78% w/w Sorbolac400

NGIs were performed on the blends and the results are set out below. Device and capsule retention were again low in these dispersion tests (> 10%)

| **Formulation** | **FPF (ED)** | **FPF (ED)** |
|---|---|---|
| 20:5:75 | 80% | 74% |
| 20:2:78 | 78% | 70% |

### Co-Jet Milled Clomipramine hydrochloride Formulations in Aspirair

Clomipramine hydrochloride was obtained in powdered form. Force control agents leucine and magnesium stearate were used.

Twelve formulations were produced from the original powder, using the Hosokawa AS50 jet mill. Either the pure drug was passed through the mill or a blend of drug with 5% w/w of a force control agent added. The mill was used with a range of parameters. Primarily, these were injector air pressure, grinding air pressure and powder feed rate.

Formulation 14: The pure clomipramine hydrochloride was passed through the microniser three times, each time with an injector air pressure of 8 bar, grinding air pressure of 1.5 bar and powder feed rate of ∼1g/min. Malvern (dry powder) particle size measurement gave a d(50) of 1.2µm.

Formulation 15: Formulation 14 was pre-blended in a pestle with a spatula with 5% micronised 1-leucine. This blend was further micronised with an injector air pressure of 8 bar, grinding air pressure of 1.5 bar and powder feed rate of ∼1g/min. Malvern (dry powder) particle size measurement gave a d(50) of 1.2µm.

Formulation 16: The pure clomipramine hydrochloride was micronised with an injector air pressure of 7 bar, grinding air pressure of 5 bar and powder feed rate of ∼10g/min. Malvern (dry powder) particle size measurement gave a d(50) of 1.0µm.

Formulation 17: The pure clomipramine hydrochloride was micronised with an injector air pressure of 7 bar, grinding air pressure of 5 bar and powder feed rate of ∼10g/min. This micronised clomipramine hydrochloride was pre-blended in a pestle with a spatula with 5% micronised 1-leucine. This blend was then micronised with an injector air pressure of 7 bar, grinding air pressure of 5 bar and powder feed rate of ∼10g/min. Malvern (dry powder) particle size measurement gave a d(50) of 0.95µm.

Formulation 18: The pure clomipramine hydrochloride was pre-blended in a pestle with a spatula with 5% magnesium stearate. This blend was micronised with an injector air pressure of 7 bar, grinding air pressure of 5 bar and powder feed rate of ∼10g/min. Malvern (dry powder) particle size measurement gave a d(50) of 0.95µm.

Formulation 19: The pure clomipramine hydrochloride was micronised with an injector air pressure of 7 bar, grinding air pressure of 1 bar and powder feed rate of ∼1g/min. Malvern (dry powder) particle size measurement gave a d(50) of 1.8µm.

This pre-micronised clomipramine hydrochloride was then blended in a pestle with a spatula with 5% micronised 1-leucine. This blend was then micronised with an injector air pressure of 7 bar, grinding air pressure of 1 bar and powder feed rate of ∼1g/min. Malvern (dry powder) particle size measurement gave a d(50) of 1.38µm.

Formulation 20: The pure clomipramine hydrochloride was micronised with an injector air pressure of 7 bar, grinding air pressure of 1 bar and powder feed rate of ∼10g/min. Malvern (dry powder) particle size measurement gave a d(50) of 3.5µm.

This pre-micronised clomipramine hydrochloride was then blended in a pestle with a spatula with 5% micronised 1-leucine. This blend was then micronised with an injector air pressure of 7 bar, grinding air pressure of 1 bar and powder feed rate of ∼10g/min. Malvern (dry powder) particle size measurement gave a d(50) of 2.0µm.

Formulation 21: The pure clomipramine hydrochloride was micronised with an injector air pressure of 7 bar, grinding air pressure of 3 bar and powder feed rate of ∼1g/min. Malvern (dry powder) particle size measurement gave a d(50) of 1.2µm.

This pre-micronised clomipramine hydrochloride was then blended in a pestle with a spatula with 5% micronised 1-leucine. This blend was then micronised with an injector air pressure of 7 bar, grinding air pressure of 3 bar and powder feed rate of ∼1g/min. Malvern (dry powder) particle size measurement gave a d(50) of 0.99µm.

Formulation 22 The pure clomipramine hydrochloride was micronised with an injector air pressure of 7 bar, grinding air pressure of 3 bar and powder feed rate of ∼10g/min. Malvern (dry powder) particle size measurement gave a d(50) of 1.6µm.

This pre-micronised clomipramine hydrochloride was then blended in a pestle with a spatula with 5% micronised 1-leucine. This blend was then micronised with an injector air pressure of 7 bar, grinding air pressure of 3 bar and powder feed rate of ∼10g/min. Malvern (dry powder) particle size measurement gave a d(50) of 1.1 µm.

Formulation 23: The clomipramine hydrochloride was pre-blended in a pestle with a spatula with 5% micronised 1-leucine. This blend was micronised with an injector air pressure of 7 bar, grinding air pressure of 5 bar and powder feed rate of ∼10g/min. Malvern (dry powder) particle size measurement gave a d(50) of 1.8µm.

Formulation 24: The pure clomipramine hydrochloride was micronised with an injector air pressure of 7 bar, grinding air pressure of 5 bar and powder feed rate of ∼10g/min.

This pre-micronised clomipramine hydrochloride was then blended in a pestle with a spatula with 5% magnesium stearate. This blend was then micronised with an injector air pressure of 7 bar, grinding air pressure of 1 bar and powder feed rate of ∼10g/min. Malvern (dry powder) particle size measurement gave a d(50) of 1.38µm.

Formulation 25: Formulation 24 was then processed in the Hosokawa MechanoFusion Minikit with 1mm compression gap for 10 minutes. Malvern (dry powder) particle size measurement gave a d(50) of 1.39µm.

### Particle size distributions

The Malvern particle size distributions show that clomipramine hydrochloride micronised very readily to small particle sizes. For example, Formulation 16 micronised to 1.0µm with one pass at the relatively high grinding pressure of 5 bar and the higher powder feed rate of 10g/min.

Reducing the grinding pressure, for example to 1 bar as with Formulation 19 interim powder, resulted in larger particles (d(50) ∼1.8µm). Intermediate grinding pressure (3 bar) gave an intermediate particle size distribution (d(50) ∼1.2µm as for Formulation 21 interim powder).

Similarly, increasing powder feed rate, for example from 1 to 10 g/min, resulted in larger particles, as can be seen by comparing d(50)s for Formulations 19 and 20.

The addition of an additive material, for example leucine as in Formulation 23, appeared to reduce the milling efficiency. However, this change may have been caused by the concomitant improvement in flowability of the original drug powder leading to a small but significant increase in the powder feed rate into the mill. It was observed in other studies that milling efficiency was increasingly sensitive to this powder feed rate as it increased above 10g/min.

It appeared possible from this series of examples to design the milling parameters to select a particular d(50). For example, a d(50) of ∼1.4 could be obtained either by repeated low pressure milling and low feed rate (Formulation 19) or by a mix of higher and lower pressure milling at a higher feed rate (Formulation 25).

### Aspirair dispersion performance

Approximately 2mg of each formulation was then loaded and sealed into a foil blister. This was then fired from an Aspirair device into a Next Generation Impactor with air flow set at 60 1/min. The performance data are summarised in Tables 36, 37 and 38.

**Table 36**

| **Formulation** | **MD (mg)** | **DD (mg)** | **FPD (mg)** | **MMAD** |
|---|---|---|---|---|
| **14** (pure drug, jet milled at 8/1.5 bar) | 1.64 | 1.19 | 1.05 | 1.53 |
| **15** (5% leucine, jet-milled at 8/1.5 bar) | 1.55 | 1.32 | 1.19 | 1.68 |
| **16** (pure drug, jet-milled at 7/5 bar) | 2.414 | 1.832 | 1.493 | 1.80 |
| **17** (5% leucine, jet-milled at 7/5 bar) | 2.120 | 1.624 | 1.474 | 1.52 |
| **18** (5% MgSt, jet-milled at 7/5 bar) | 1.737 | 1.519 | 1.390 | 1.44 |
| **19** (5% leucine, jet-milled at 7/1 bar) | 2.031 | 1.839 | 1.550 | 1.90 |
| **20** (5% leucine, jet-milled at 7/1 bar) | 1.821 | 1.685 | 1.071 | 2.44 |
| **21** (5% leucine, jet-milled at 7/3 bar) | 1.846 | 1.523 | 1.437 | 1.61 |
| **22** (5% leucine, jet-milled at 7/3 bar) | 2.213 | 1.940 | 1.733 | 1.72 |
| **23** (5% leucine, single pass at 7/5 bar) | 1.696 | 1.557 | 1.147 | 2.13 |
| **24** (5% MgSt, jet-milled at 7/5 bar & Mechano-Fused) | 1.743 | 1.542 | 1.274 | 1.82 |
| 25 (5% MgSt, jet-milled at 7/5 bar) | 1.677 | 1.570 | 1.351 | 1.72 |

**Table 37**

| **Formulation** | **FPF % (<5µm)** | **FPF % (<3µm)** | **FPF % (<2µm)** | **FPF % (<1µm)** |
|---|---|---|---|---|
| **14** (pure drug, jet milled at 8/1.5 bar) | 88 | 83 | 65 | 21 |
| **15** (5% leucine, jet-milled at 8/1.5 bar) | 90 | 82 | 60 | 17 |
| **16** (pure drug, jet-milled at 7/5 bar) | 82 | 71 | 51 | 14 |
| **17** (5% leucine, jet-milled at 7/5 bar) | 91 | 85 | 68 | 21 |
| **18** (5% MgSt, jet-milled at 7/5 bar) | 91 | 90 | 73 | 20 |
| **19** (5% leucine, jet-milled at 7/1 bar) | 84 | 74 | 48 | 10 |
| **20** (5% leucine, jet-milled at 7/1 bar) | 64 | 46 | 28 | 6 |
| **21** (5% leucine, jet-milled at 7/3 bar) | 94 | 88 | 67 | 14 |
| **22** (5% leucine, jet-milled at 7/3 bar) | 89 | 80 | 56 | 14 |
| **23** (5% leucine, single pass at 7/5 bar) | 74 | 57 | 37 | 9 |
| **24** (5% MgSt, jet-milled at 7/5 bar & Mechano-Fused) | 83 | 68 | 47 | 15 |
| **25** (5% MgSt, jet-milled at 7/5 bar) | 86 | 74 | 53 | 21 |

**Table 38**

| **Formulation** | **Recovery %** | **Throat %** | **Blister %** | **Device %** |
|---|---|---|---|---|
| **14** (pure drug, jet milled at 8/1.5 bar) | 82 | 8 | 1 | 26 |
| **15** (5% leucine, jet-milled at 8/1.5 bar) | 81 | 7 | 0 | 15 |
| **16** (pure drug, jet-milled at 7/5 bar) | 121 | 10 | 3 | 21 |
| **17** (5% leucine, jet-milled at 7/5 bar) | 106 | 5 | 1 | 23 |
| **18** (5% MgSt, jet-milled at 7/5 bar) | 91 | 6 | 0 | 12 |
| **19** (5% leucine, jet-milled at 7/1 bar) | 107 | 10.6 | 1.3 | 8.2 |
| **20** (5% leucine, jet-milled at 7/1 bar) | 96 | 24 | 1.3 | 6.1 |
| **21** (5% leucine, jet-milled at 7/3 bar) | 97 | 3 | 0.6 | 16.9 |
| **22** (5% leucine, jet-milled at 7/3 bar) | 116 | 7 | 0.6 | 16.9 |
| **23** (5% leucine, single pass at 7/5 bar) | 87 | 18 | 2 | 6 |
| **24** (5% MgSt, jet-milled at 7/5 bar & Mechano-Fused) | 92 | 14 | 1 | 10 |
| **25** (5% MgSt, jet-milled at 7/5 bar) | 87 | 10 | 1 | 6 |

The device retention appeared high (above 20%) where pure drug was used, and especially increased with small particle sizes (especially 1µm and below): for example Formulations 14 and 16 had high drug retention. Device retention was lower with use of magnesium stearate, for example as with Formulation 18 where device retention was 12% despite a d(50) of 0.95µm. Device retention was also reduced below 20% when leucine was used in combination with a particle size above 1µm, for example with Formulation 22.

Throat deposition was reduced proportionately as particle size was reduced. High throat deposition (>20%) occurs with particle size d(50)>2µm: e.g. Formulation 20. Throat deposition of below 10% was seen for particle sizes below 1µm. The reduced inertial behaviour of the smaller particles may well contribute to this observation. However, as noted above, device retention tended to be greater for such small particles.

It is argued that as particle size was reduced, increased adhesiveness and cohesiveness results in increased device retention. This adhesiveness and cohesiveness and hence device retention can be reduced by addition of force control agents, attached to the drug particle surface (or drug and excipient particle surfaces, as appropriate). As argued previously for the apomorphine and clobozam examples, and demonstrated by the video study, in Aspirair it is believed that a level of adhesiveness and cohesiveness is desirable to prolong lifetime in the vortex, yielding a slower plume, but adhesiveness and cohesiveness should not be so high as to result in high device retention. Consequently a balance of particle size, adhesiveness and cohesiveness is required to achieve an optimum performance in Aspirair. The examples contained herein indicate how such a balance may be achieved. This balance may require modifying for each particular different material characteristic.

Single step co-milling with a force control agent appears effective in some examples such as Formulation 18. Multiple stage processing may be more effective, for example, where the conditions are selected to achieve particularly desirable effects. For example, first stage high pressure milling of pure drug may be used to produce the required size distribution (i.e. ∼1.4µm), and a second stage lower pressure co-milling used to mix in the force control agent, whereby better mixing is achieved without milling and with reduced segregation of components in the mill. Such is shown in Formulation 25, where a combination of both relatively low throat deposition and low device retention are achieved.

The optimum amount of additive material will depend on the chemical composition and other properties of the additive material and upon the nature of the active material and/or excipient material, if present. In general, the amount of additive material in the composite active particles will be not more than 60% by weight, based on the weight of the active material and any excipient material. However, it is thought that for most additive materials the amount of additive material should be in the range of 40% to 0.25%, preferably 30% to 0.5%, more preferably 20% to 2%, based on the total weight of the additive material and the active material being milled. In general, the amount of additive material is at least 0.01% by weight based on the weight of the active material.

Clearly, many different designs of jet mills exist and any of these may be used in the present invention. For example, in addition to the AS50 Spiral jet mill and the MC50 Hosakawa Micron used in the experiments discussed above, one can also use other spiral jet mills, pancake jet mills or opposed fluid bed jet mills. The feed rate for the jet mills will depend on their size. Small spiral jet mills might use a feed rate of, for example, 1 to 2g per minute, whilst industrial scale mills will have a feed rate in the order of kilograms per hour.

The properties of the co-jet milled particles produced using the present invention may, to an extent, be tailored or adjusted by making changes to the jet milling apparatus. For example, the degree of particle coating and particle size reduction may be adjusted by changing the number of jets which are used in the apparatus, and/or by adjusting their orientation, that is, the angles at which they are positioned.

### Conclusions

The improvements in the dry powder inhaler devices and in the dry powder formulations mean that the desired dose efficiency can be achieved. The following tests demonstrate this.

The in-vitro testing was performed using the Aspirair device and using formulations prepared as follows.

120 g of Respitose SV003 lactose (45 to 63µm sieve fraction) and 30 g of micronised apomorphine hydrochloride and were combined into the mixing bowl of a Glen Creston GrindoMix high shear blender. The drug was sandwiched between Respitose layers. The material was processed at a setting of 2000rpm for 5 minutes. The blend was screened through a 250µm sieve.

Content uniformity was assessed by taking 10 samples of 3mg from the bulk powder. The formulation contained a mean drug content of 20.8%, with a relative standard deviation of 1.97%.

2mg of powder was filled into 25 Aspirair foil blisters. 5 blisters were fired from an Aspirair device into a 60 litre per minute Andersen Cascade Impactor (ACI), with air flow set at 60 litres per minute. The Aspirair was fired with 15ml of reservoir air at 1.5 bar. This was repeated 5 times and the results are summarised in Tables 39 and 40.

**Table 39**

| **Formulation** | **MD (mg)** | **DD (mg)** | **FPD (mg) (<5µm)** | **FPF(MD) % (<5µm)** | **FPF(ED) % (<5µm)** |
|---|---|---|---|---|---|
| | 0.38 | 0.36 | 0.29 | 75 | 81 |
| | 0.38 | 0.35 | 0.28 | 74 | 80 |
| | 0.40 | 0.37 | 0.30 | 75 | 81 |
| | 0.39 | 0.36 | 0.29 | 74 | 80 |
| | 0.38 | 0.35 | 0.29 | 75 | 82 |
| **Mean** | 0.39 | 0.36 | 0.29 | 75 | 81 |

**Table 40**

| **Formulation** | **FPF(ED) % (<3µm)** | **FPF(ED) % (<2µm)** | **MMAD** | **Blister retention (%)** | **Device retention (%)** |
|---|---|---|---|---|---|
| | 75 | 54 | **1.70** | 2 | 6 |
| | 74 | 52 | **1.73** | 2 | 6 |
| | 74 | 53 | **1.72** | 2 | 6 |
| | 74 | 55 | **1.66** | 2 | 6 |
| | 75 | 55 | **1.68** | 2 | 6 |
| **Mean** | 74 | 54 | **1.70** | 2% | 6% |

The formulations exhibited exceptional fine particle fractions of emitted dose and of metered dose. Also the performance is very consistent, between all 5 repeated tests.

In a further study, also using the CL1 Aspirair device, the following formulation was tested. Respitose SV003 lactose (45 to 63µm sieve fraction) and micronised salbutamol sulphate were combined in the ratio 60:40.

1mg of powder was filled into 15 Aspirair foil blisters. 5 blisters were fired from an Aspirair device into a Next Generation Impactor with air flow set at 60 litres per minute. The Aspirair was fired with 15ml of reservoir air at 1.5 bar. This was repeated 3 times. The results are summarised in Tables 41 and 42.

**Table 41**

| **NGI** | **MD (µg)** | **ED (µg)** | **FPD >5µm (µg)** | **FPF(MD)% >5µm** | **MMAD** |
|---|---|---|---|---|---|
| 1 | 484 | 470 | 397 | 82 | 1.80 |
| 2 | 376 | 367 | 328 | 87 | 1.78 |
| 3 | 404 | 390 | 350 | 87 | 1.74 |

**Table 42**

| **NGI** | **FPF(ED)% >5µm** | **FPF(ED)% >3µm** | **FPF(ED)% >2µm** | **FPF(ED)% >1µm** |
|---|---|---|---|---|
| 1 | 85 | 73 | 53 | 21 |
| 2 | 89 | 78 | 55 | 17 |
| 3 | 90 | 79 | 56 | 19 |

Once again, the formulations exhibited exceptional and reproducible fine particle fractions of emitted dose and of metered dose.

### Example 1: Inhalation testing

The above referenced blisters containing the 100 and 200 microgram apomorphine-lactose formulations were subjected to testing using an Aspirair prototype inhaler.

In order to obtain the inhalation data described below, the inhaler device was used in conjunction with three instruments, a Multi-Stage Liquid Impinger (MSLI) (U.S.P. 26, Chapter 601, Apparatus 4 (2003), an Anderson Cascade Impactor (ACI) (U.S.P. 26, Chapter 601, Apparatus 3 (2003), and a Dosage Unit Sampling Apparatus (DUSA) (U.S.P. 26, Chapter 601, Apparatus B (2003). Each of these devices has an input for receiving the mouthpiece of the inhaler.

The DUSA is used to measure the total amount of drug which leaves the inhaler. With data from this device, the metered and delivered dose is obtained. The delivered dose is defined as the amount of drug that leaves the inhaler. This includes the amount of drug in the throat of the DUSA device, in the measuring section of the DUSA device and the subsequent filters of the DUSA device. It does not include drug left in the blister or other areas of the inhaler, and does not account for drug "lost" in the measuring process of the DUSA device. The metered dose includes all of the drug which leaves the blister.

The MSLI is a device for estimating deep lung delivery of a dry powder formulation. The MSLI includes a five stage cascade impactor which can be used for determining the particle size (aerodynamic size distribution) of Dry Powder Inhalers (DPIs) in accordance with USP 26, Chapter 601, Apparatus 4 (2003) and in accordance with the European Pharmacopoeia, Method 5.2.9.18, Apparatus C, Supplement 2000.

The ACI is another device for estimating deep lung delivery of a dry powder formulation. The ACI is multi-stage cascade impactor which can be used for determining the particle size (aerodynamic size distribution) of dry powder inhalers (DPI) in accordance with USP 26, Chapter 601, Apparatus 3 (2003).

As described below, the MSLI and the ACI testing devices can be used to determine, *inter alia,* the fine particle dose (FPD), i.e. the amount of drug, e.g., in micrograms, that is measured in the sections of the testing device which correlates with deep lung delivery and the fine particle fraction (FPF), i.e. the percentage of the metered dose which is measured in the sections of the testing device which correlates with deep lung delivery.

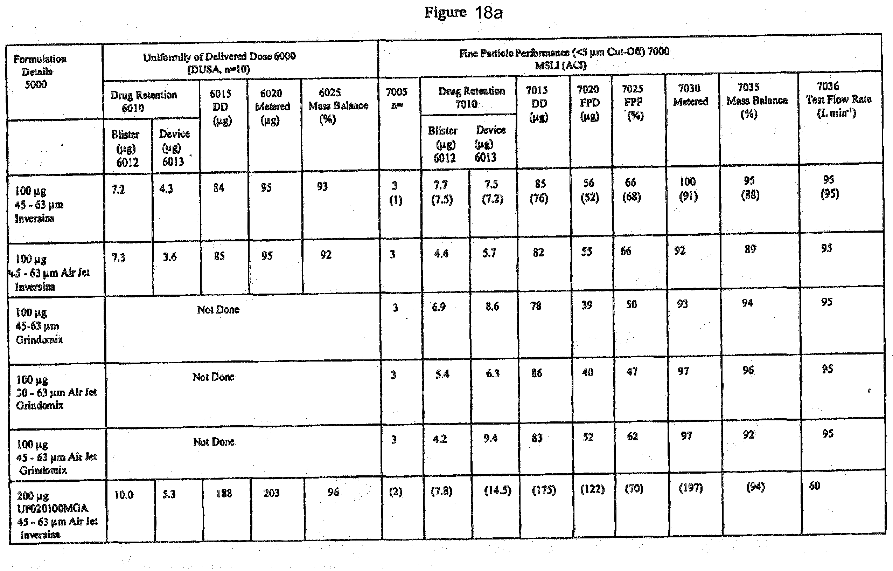

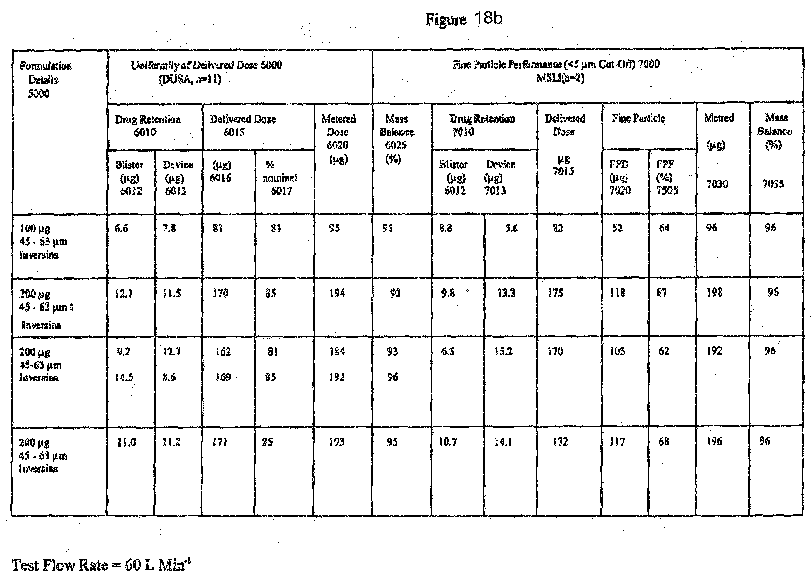

Figures 18A and 18B illustrate the results of tests performed on the apomorphine-lactose formulation prepared as follows. Apomorphine hydrochloride was obtained from Macfarlan Smith Ltd, and was micronised according to the following product specification: ≥99.9% by mass <10µm, based upon a laser diffraction analysis. Actual typical results of the laser fraction analysis were as follows: d₁₀ <1µm, d₅₀: 1-3µm; d₉₀<6µm, wherein d₁₀ d₅₀ d₉₀ refer to the diameter of 10%, 50%, and 90% of the analysed apomorphine hydrochloride. The apomorphine hydrochloride was micronised with nitrogen, (rather than the commonly employed air) to prevent oxidative degradation. The FPD, FPF and MMAD values were generated from the MSLI and ACI data using the Copley Inhaler Data Analysis Software (CITDAS) V1.12. In Figure 18A, data is shown for six formulations, which are identified in column 5000. Figure 18B provides data for an additional four formulations. In each Figure, the test data for the formulations is divided into two types: data relating to uniformity of the delivered dose for the formulations (column 6000) and data relating to fine particle size performance of the formulations (column 7000).

Referring to Figure 18A, the first five formulations listed in column 5000 include 3mg of the 100 microgram formulation prepared according to the following method 'B'. A sieved fraction of Respitose SV003 (DMV International Pharma, The Netherlands) lactose is manufactured by passing bulk material through a 63µm sieve. This material is then sieved through a 45µm screen and the retained material is collected. The resultant lactose has a volume weighted mean of from about 50 to about 55µm, a d₁₀ of from about 4 to about 10µm, a d₅₀ of from about 50 to about 55µm, and a d₉₀ of from about 85 to about 95µm wherein d₁₀ d₅₀ d₉₀ refer to the diameter of 10%, 50%, and 90% of the analysed lactose.

72.5 grams of this lactose were placed into a metal mixing vessel of a suitable mixer. 5 grams of the micronised apomorphine hydrochloride were then added. An additional 72.5 grams of the lactose were then added to the mixing vessel, and the resultant mixture was tumbled for 15 minutes. The resultant blend was then passed through a 150µm screen. The screened blend (i.e. the portion of the blend that passed through the screen) was then reblended for 15 minutes.

The mixer used was an Inversina Variable Speed Tumbler Mixer, which is a low shear mixer distributed by Christison Scientific Equipment Ltd of Gateshead, U.K.. In other batches, the mixer used was a Retsch Grindomix mixer is a higher shear mixer which is also distributed by Christison Scientific Equipment Ltd. Disaggregation was shown to be sensitive to the intensity of the mixing process but a consistent fine particle fraction (about 60%) was obtained using a low shear mixer equipped with a metal vessel such as the Inversina mixer.

The sixth formulation listed in Figure 18A includes 3mg of the 200 microgram formulation prepared according to the following method 'B'. 70 grams of the lactose described above were placed into a metal mixing vessel of a suitable mixer. 10 grams of the micronised apomorphine hydrochloride were then added. An additional 70 grams of the lactose were then added to the mixing vessel, and the resultant mixture was tumbled for 15 minutes. The resultant blend was then passed through a 150µm screen. The screened blend (i.e. the portion of the blend that passed through the screen) was then reblended for 15 minutes.

The particle size distribution of the apomorphine-lactose powder, as determined by an Andersen Cascade Impactor (U.S.P. 26, Chapter 601, Apparatus 3 (2003)), showed that the drug particles were well dispersed. In particular, the particle size distribution for a 200µg dose was as follows:

| | |
|---|---|
| Fine particle dose (<5µm) | 117µg |
| Ultrafine particle dose (<2.5µm) | 80µg |
| MMAD (Mass Median Aerodynamic Diameter) | 1.94µm |

The first, second, and sixth formulation listings in 5000 of Figure 18A contain the notation "Inversina" to indicate that the mixer used was the Inversina Mixer, and the third, fourth, and fifth formulation listing contain the notation "Grindomix" to indicate that the mixer used was the Grindomix Mixer. The second and fourth formulations listed also contain the notation "Air Jet" to indicate that for these formulations the lactose was sieved with an Air Jet Sieve which applies a vacuum to the screen sieve apparatus, rather than a conventional screen sieve (which was used for the first third, fifth, and sixth formulations listed). The fifth formulation listed also contains the notation "20-30µm Extra Fine" to indicate that the lactose for this formulation was screen sieved through 20µm and 30µm screens.

In section 6000 of Figure 18A, the DUSA apparatus described above is used to provide data for the formulations regarding the drug retention in the blister (6012), the drug retention in the inhaler (6013), the delivered dose (6015), the metered dose (6020), and the mass balance percentage (6025). The notation n=10 indicates that the inhaler and DUSA apparatus was fired 10 times for each of the three formulations for which DUSA data is listed. The data listed in section 6000 is an average of the 10 firings.

In section 7000 of Figure 18B, the fine particle performance is measured with two different devices, the MSLI and the ACI. Data for the ACI, where available, is indicated in parenthesis (). In any event, the data provided in section 7000 is for particles having a particle size diameter of less than 5µm (referred to in this discussion as "fine particles"). As such, column 7012 provides the fine particle drug retention in the blister, column 7013 provides the fine particle drug retention in the inhaler, column 7015 provides the amount of fine particles in the delivered dose, column 7020 provides the FPD for the formulation, column 7025 provides the FPF for the formulation, column 7015 provides the amount of fine particles in the metered dose, column 7035 provides the mass balance percentage for the formulations in the MSLI (ACI) tests, and column 7036 provides the test flow rate for the formulations. Column 7005 indicates that the number of times the inhaler and MSLI (or ACI) apparatus were fired, and the data listed is an average of the "n" firings.

Figure 18B is similar to Figure 18A, with similar items bearing identical reference numbers. The first formulation listed in column 5000 include 3mg of the 100 microgram formulation prepared according to the above method 'A', the remaining four formulations include 3mg of the 200 microgram formulation according to the above method 'B', and all of the formulations were made with the Inversina Mixer, and were sieved with 43 and 63µm screens. The DUSA data in column 6000 was obtained in the same manner as in Figure 18A, except that n=11. All of the fine particle performance data in section 7000 was obtained using the ACI apparatus with n= 2, and a flow rate of 60 L min⁻¹.

As illustrated in Figures 18A and 18B, when the formulations were mixed using the low shear Inversina mixer, the fine particle fraction (FPF) ranged from a low of 62% to a high of 70%, and the percent delivered dose ranged from a low of 81% to a high of 94%. The formulations made with the higher shear Grindomix mixer exhibited a fine particle fraction of from 47% to 50% for formulations including the 43-63µm lactose. The formulation made with the high shear Grindomix mixer and with lactose sieved at 20 and 30µm exhibited an increased fine particle fraction of 62%.

### Example 2: 400µg apomorphine hydrochloride capsule for use in Cyclohaler

Five 400µg apomorphine hydrochloride capsules were prepared and tested in a Cyclohaler inhaler (trade mark) (available from Miat) in an ACI (U.S.P. 26, Chapter 601, Apparatus 3) configured for operation at 100 l.min⁻¹. Each capsule had a fill weight of 25mg, and included the following components:

| **Component** | **Weight (g)** | **Weight % (w/w)** |
|---|---|---|
| Pharmatose 150M (DMV Pharma) | 127.725 | 85.15 |
| Sorbolac 400 (Meggle Pharma) | 12.375 | 8.25 |
| Micronised Leucine | 7.500 | 5.00 |
| Apomorphine Hydrochloride (d₅₀ =1.453µm) | 2.400 | 1.60 |

In this regard, Pharmatose 150M, available from DMV Pharma, comprises lactose with the following particle size distribution (according to DMV Pharma literature): 100% less than 315µm, at least 85% less than 150µm, at least 70% less than 100µm, and at least 50% less than 45µm. Sorbolac 400, available from Meggle Pharma comprises lactose with the following particle size distribution (according to Meggle Pharma literature): 100% less than 100µm, at least 99% less than 63µm, and at least 96% less than 32µm.

### Preparation of Pre-blend

The Pharmatose, Sorbolac and leucine were layered in the mixing bowl so that the leucine was sandwiched between the Sorbolac, which in turn was sandwiched between the Pharmatose. The powders were blended for 60 seconds at 2000rpm using the Retsch Grindomix High Shear Mixer described above. The pre-blend was rested for 1 hour before further use.

### Preparation of Final Blend

The apomorphine hydrochloride was sandwiched between the pre-blend in the mixing bowl. Blending was carried out for 10 minutes at 2000rpm using the Grindomix mixer. The blend was then passed through a 212µm sieve.

Thereafter, the final blend was placed in capsules, each capsule having a fill weight of 25mg. The capsules were then placed in a Cyclohaler and tested in an ACI

(U.S.P. 26, Chapter 601, Apparatus 3), with the data analysed via the CITDAS described above, providing the following results:

| | |
|---|---|
| Delivered Dose (%) (100*Delivered Dose/Total Dose) | 81% |
| %Fine Particle Fraction (percent of the delivered dose <5µm) | 67% |
| %Fine Particle Dose (percent of the total dose <5µm) | 55% |
| MMAD | 2.3µm |
| Fine Particle Dose | 220µg |
| %Ultrafine Particle Dose (percent of the total dose <3µm) | 44% |
| Ultrafine Particle Dose | 175µg |
| Ultrafine Particle Fraction | 53% |

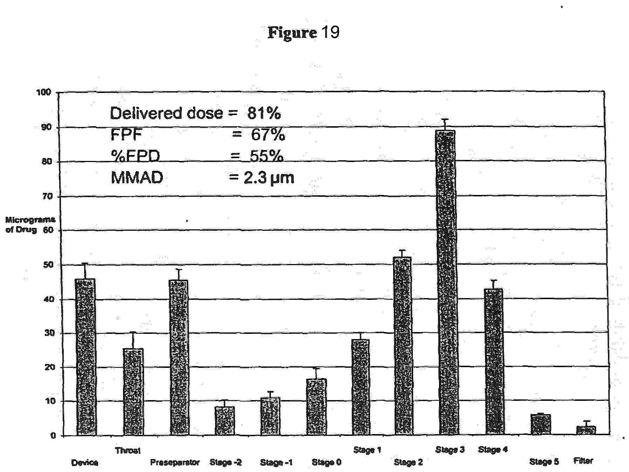

Figure 19 illustrates the average amount (in micrograms) of drug that was delivered to each of the components of the ACI, and retained in the device. Thus, for example, the ultrafine particle dose can be produced from this data by the CITDAS package.

### Example 3: 400µg apomorphine hydrochloride 2mg blister

Five 400µg apomorphine hydrochloride blisters were prepared and tested in the inhaler of Example 1 in an ACI (USP 26, Chapter 601, Apparatus 3) configured for operation at 60 1.min⁻¹. Each blister had a fill weight of 2mg, and included the following components:

| **Component** | **Weight (g)** | **Weight % (w/w)** |
|---|---|---|
| Respitose 45-63µm sieve | 120 | 80 |
| Apomorphine Hydrochloride (d₅₀ = 1.453µm) | 30 | 20 |

The apomorphine hydrochloride was sandwiched between the Respitose in the mixing bowl as generally described in methods 'A' and 'B'. The powders were blended for 5 minutes at 2000rpm using the Grindomix mixer. The blend was then passed through a 212µm sieve. Thereafter, the blend was placed in blister, each blister having a fill weight of 2mg. The blisters were then placed in the inhaler of Example 1 and tested in an ACI (U.S.P. 26, Chapter 601, Apparatus 3), with the data analysed via the CITDAS described above, providing the following results:

| | |
|---|---|
| Delivered Dose (%) (100*Delivered Dose/Total Dose) | 89% |
| %Fine Particle Fraction (percent of the delivered dose <5µm) | 81% |
| %Fine Particle Dose (percent of the total dose <5µm) | 72% |
| MMAD | 1.70µm |
| Fine Particle Dose | 288µg |
| %Ultrafine Particle Dose (percent of the total dose <3µm) | 67% |
| Ultrafine Particle Dose | 266µg |
| %Ultrafine Particle Fraction (percent of the delivered dose <3µm) | 75% |

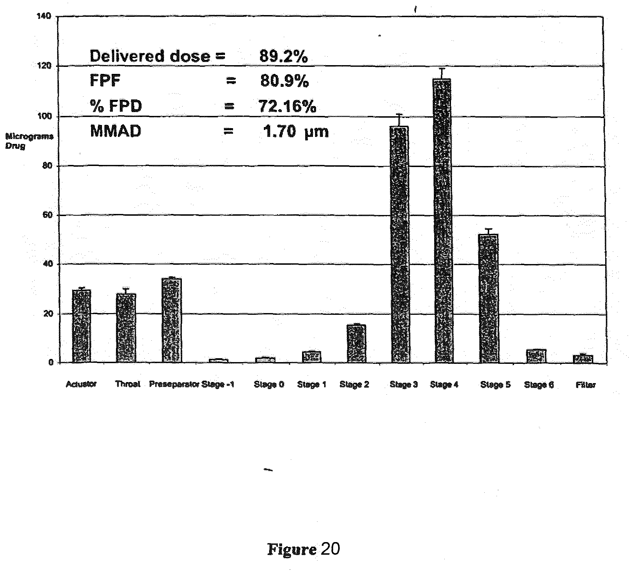

Figure 20 illustrates the average amount (in micrograms) of drug that was delivered to the components of the ACI, and left in the device. Thus, for example, the ultrafine particle dose can be produced from this data using the CITDAS package.

It should be noted that the MMAD of 1.70µm generated from the ACI data is remarkably fine, and very close to the median diameter determined by laser light diffraction, for this batch of apomorphine hydrochloride (1.453µm). This indicates that the inhaler is efficiently reducing the drug to, or close to, its primary particles, rather than agglomerate. This is highly unusual for an inhaler. For example, when the same batch of apomorphine hydrochloride (i.e., in particle size) was delivered with the Cyclohaler of Example 2, a larger MMAD of 2.3µm was measured, indicating that this formulation and device was not as efficient at eliminating agglomerates.

When compared with the formulation and inhaler of Example 2, the formulation and inhaler of Example 3 also provided a superior delivered dose (89.2% vs. 81%), fine particle fraction (81% vs. 67%), %fine particle dose (72% vs. 55%) and %ultrafine particle dose (67% vs. 44%).

It is also apparent from the above data that the formulation and inhaler of Example 3 produces an ultrafine particle fraction (<3µm) of more than 70%. While a fine particle fraction (<5µm) can be considered acceptable for local delivery, it is believed that for systemic delivery, even finer particles are needed, because the drug must reach the alveoli to be absorbed into the bloodstream. As such an ultrafine particle fraction in excess of 70% is particularly advantageous.

### Example 4: Preparation of MechanoFused formulation for use in passive device

20g of a mix comprising 20%micronised clomipramine, 78% Sorbolac 400 lactose and 2% magnesium stearate were weighed into the Hosokawa AMS-MINI MechanoFusion system via a funnel attached to the largest port in the lid with the equipment running at 3.5%. The port was sealed and the cooling water switched on. The equipment was run at 20% for 5 minutes followed by 80% for 10 minutes. The equipment was switched off, dismantled and the resulting formulation recovered mechanically.

20mg of the collected powder formulation was filled into size 3 capsules and fired from a Miat Monhaler into an NGI. The FPF measured was greater than 70%.

In light of the foregoing data and examples, one can see that excellent performance of the powder formulations according to the present disclosure (defined in terms of FPF(ED) and FPF(MD) at 5µm, 3µm and 2µm) is achieved in vitro. What is more, this high performance also leads to excellent in vivo performance, including achieving faster peak blood levels than alternative systems. Indeed, peak blood levels may be achieved within 1 to 10 minutes from administration when using the present disclosure. This, in turn, leads to a faster onset of the clinical effect than is observed with alternative systems. Indeed, the onset may be 2, 3, 5, or even 10 times faster when using the present disclosure.

Another very important advantage of the system disclosed herein is the consistency of the high performance. The data set out above shows that the excellent performance is repeatable with very low variability. One of the many benefits of such consistency is that it can also lead to reduction in adverse side effects experienced, as it will allow one to administer a smaller total dose than is possible when relying upon conventional inhaler efficiency or other routes of administration. In particular, it allows one to target specific dosing windows wherein the therapeutic effect is maximised whilst causing the minimum side effects.

The system disclosed herein is extremely flexible and therefore has a vast number of applications.

The formulations may be administered using active or passive devices, as it has been identified how to tailor the formulation to the device used to dispense it, thereby overcoming some of the perceived disadvantages of passive devices where high performance is sought.

The size of the doses can vary from micrograms to tens of milligrams. The fact that dense particles may be used, in contrast to conventional thinking, means that larger doses can be administered without needing to administer large volumes of powder and the problems associated therewith.

The dry powder formulations may be pre-metered and kept in foil blisters which offer chemical and physical protection whilst not being detrimental to the overall performance. Indeed, the formulations thus packaged tend to be stable over long periods of time, which is very beneficial, especially from a commercial and economic point of view.

The milling methods used in the present disclosure for preparing the fine particles of active agent are simple and cheap compared to the complex previous attempts to engineer particles, providing practical as well as cost benefits. In addition, the spray drying methods provided herein are also capable of being carried out on a large scale, again providing practical and cost benefits.

A further benefit associated with the present disclosure is that the powder process step may be dry, which means that it does not have to involve organic solvents.

Such organic solvents are common to many of the known approaches to powder processing.

In addition, the active agents used in the present invention may be small molecules, proteins, carbohydrates or mixtures thereof.

Finally, the particles prepared as described herein are not "low density" particles, as tend to be favoured in the prior art. Rather, the jet milled and spray dried particles are made using simple processes. Previously, those skilled in the art have only reported high performance in connection with powder particles that have been prepared using fancy processing techniques such as complex spray drying, which result in low density particles. As explained above, surprisingly it is advantageous not to produce severely dimpled or wrinkled particles as these can yield low density powders, with very high voidage between particles. Such powders occupy a large volume relative to their mass as a consequence of this form, and can result in packaging problems, i.e., require much larger blisters or capsules are required to hold a given mass of powder.

Advantageously, the powders prepared according to the present invention have a tapped density of at least 0.1g/cm³ (g/cc), at least 0.2g/cm³ (g/cc), at least 0.3g/cm³ (g/cc), at least 0.4g/cm³ (g/cc) or at least 0.5g/cm³ (g/cc).

## Claims

1. A dry powder inhaler device comprising a dry powder formulation comprising a pharmaceutically active agent in the form of composite active particles, wherein upon actuation of the device, a dosing-efficiency at 5µm of at least 70% is achieved, wherein the composite active particles are prepared using a method comprising jet milling active particles in the presence of particles of additive material, wherein the additive material is a metal stearate and wherein the formulation additionally comprises carrier particles that also have additive material applied to their surfaces optionally by simple gentle blending or co- milling.

2. . A device as claimed in claim 1, wherein the additive forms a discontinuous coating on the surface of the particles of pharmaceutically active agent.

3. . A device as claimed in any one of the preceding claims, wherein the additive forms a discontinuous coating on the surface of the carrier particles.

4. . A device as claimed in any one of the preceding claims, wherein the dry powder formulation is in pre- metered doses stored in one or more foil blisters.

5. . A device as claimed in any one of the preceding claims, wherein the dry powder formulation is produced by a micronisation process.

6. . A device as claimed in any one of the preceding claims, wherein the dry powder formulation has a tapped density of more than 0.1g/cm³ (g/cc), preferably wherein the formulation has a tapped density of more than 0.2g/cm³ (g/cc), more preferably wherein the formulation has a tapped density of more than 0.5g/cm³ (g/cc.)

7. . A device as claimed in any one of the preceding claims, wherein the pharmaceutically active agent has a systemic effect following administration by pulmonary inhalation.

8. . A device as claimed in any one of the preceding claims, wherein the pharmaceutically active agent is selected from heparin, apomorphine, Glycopyrrolate, clomipramine or clobozam.

9. . A device as claimed in any one of the preceding claims, wherein the dry powder formulation is processed without the use of an organic solvent.

10. . A device as claimed in any one of the preceding claims, wherein the dry powder formulation is dry processed in the absence of any solvent.

11. . A device as claimed in any one of claims 1 to 8, wherein the carrier is lactose.

12. . A device as claimed in any one of claims 1 to 11, wherein the carrier is lactose and the additive is magnesium stearate.

13. . A device as described in any one of claims 1-12 wherein a plurality of active agents are present in the formulation.

14. . A composite active particle for use in a dry powder formulation for pulmonary inhalation, wherein the formulation is in an inhaler, and wherein the composite active particle is prepared using a method comprising jet milling active particles in the presence of particles of additive material, wherein the additive material is a metal stearate, and wherein the formulation comprises carrier particles that also have additive material applied to their surfaces optionally by simple gentle blending or co-milling.

## Patentansprüche

1. Trockenpulverinhalatorvorrichtung, umfassend eine Trockenpulverformulierung, umfassend einen pharmazeutisch aktiven Wirkstoff in Form zusammengesetzter aktiver Partikel, wobei bei Betätigung der Vorrichtung eine Dosiereffizienz bei 5µm von mindestens 70% erzielt wird, wobei die zusammengesetzten aktiven Partikel unter Anwendung eines Verfahrens hergestellt werden, das Strahlmahlen aktiver Partikel in Gegenwart von Partikeln von Zusatzstoff umfasst, wobei der Zusatzstoff ein Metallstearat ist und wobei die Formulierung zusätzlich Trägerpartikel umfasst, an deren Oberflächen ebenfalls Zusatzstoff angebracht ist, optional durch einfaches schonendes Mischen oder gemeinsames Vermahlen.

2. Vorrichtung nach Anspruch 1, wobei der Zusatzstoff eine diskontinuierliche Beschichtung auf der Oberfläche der Partikel von pharmazeutisch aktivem Wirkstoff bildet.

3. Vorrichtung nach einem der vorhergehenden Ansprüche, wobei der Zusatzstoff eine diskontinuierliche Beschichtung auf der Oberfläche der Trägerpartikel bildet.

4. Vorrichtung nach einem der vorhergehenden Ansprüche, wobei die Trockenpulverformulierung in vordosierten Dosierungen vorliegt, die in einer oder mehreren Folienblisterpackungen gelagert sind.

5. Vorrichtung nach einem der vorhergehenden Ansprüche, wobei die Trockenpulverformulierung durch einen Mikronisierungsprozess produziert wird.

6. Vorrichtung nach einem der vorhergehenden Ansprüche, wobei die Trockenpulverformulierung eine Stampfdichte von mehr als 0,1 g/cm³ (g/ccm) aufweist, wobei die Formulierung bevorzugt eine Stampfdichte von mehr als 0,2 g/cm³ (g/ccm) aufweist, wobei die Formulierung noch bevorzugter eine Stampfdichte von mehr als 0,5 g/cm³ (g/ccm) aufweist.

7. Vorrichtung nach einem der vorhergehenden Ansprüche, wobei der pharmazeutisch aktive Wirkstoff nach Verabreichung durch pulmonale Inhalation eine systemische Wirkung hat.

8. Vorrichtung nach einem der vorhergehenden Ansprüche, wobei der pharmazeutisch aktive Wirkstoff aus Heparin, Apomorphin, Glycopyrrolat, Clomipramin oder Clobozam ausgewählt ist.

9. Vorrichtung nach einem der vorhergehenden Ansprüche, wobei die Trockenpulverformulierung ohne Anwendung eines organischen Lösungsmittels verarbeitet wird.

10. Vorrichtung nach einem der vorhergehenden Ansprüche, wobei die Trockenpulverformulierung in Abwesenheit gleich welchen Lösungsmittels trocken verarbeitet wird.

11. Vorrichtung nach einem der Ansprüche 1 bis 8, wobei der Träger Laktose ist.

12. Vorrichtung nach einem der Ansprüche 1 bis 11, wobei der Träger Laktose ist und der Zusatzstoff Magnesiumstearat ist.

13. Vorrichtung, wie in einem der Ansprüche 1-12 beschrieben, wobei eine Vielzahl aktiver Wirkstoffe in der Formulierung vorhanden ist.

14. Zusammengesetztes aktives Partikel zur Anwendung in einer Trockenpulverformulierung zur pulmonalen Inhalation, wobei die Formulierung ein Inhalator ist, und wobei das zusammengesetzte aktive Partikel unter Anwendung eines Verfahrens hergestellt wird, das Strahlmahlen aktiver Partikel in Gegenwart von Partikeln von Zusatzstoff umfasst, wobei der Zusatzstoff ein Metallstearat ist, und wobei die Formulierung Trägerpartikel umfasst, an deren Oberflächen ebenfalls Zusatzstoff angebracht ist, optional durch einfaches schonendes Mischen oder gemeinsames Vermahlen.

## Revendications

1. Dispositif d'inhalation de poudre sèche comprenant une formulation de poudre sèche comprenant un agent pharmaceutiquement actif sous la forme de particules actives composites, dans lequel lors de l'actionnement du dispositif, une efficacité de dosage à 5µm d'au moins 70% est atteinte, dans lequel les particules actives composites sont préparées en utilisant un procédé comprenant un broyage par jet des particules actives en présence de particules de matériau additif, dans lequel le matériau additif est un stéarate métallique et dans lequel la formulation comprend en outre des particules véhicules qui ont également un matériau additif appliqué sur leurs surfaces éventuellement par simple mélange léger ou co-broyage.

2. Dispositif selon la revendication 1, dans lequel l'additif forme un revêtement discontinu sur la surface des particules de l'agent pharmaceutiquement actif.

3. Dispositif selon l'une quelconque des revendications précédentes, dans lequel l'additif forme un revêtement discontinu sur la surface des particules véhicules.

4. Dispositif selon l'une quelconque des revendications précédentes, dans lequel la formulation de poudre sèche est en doses pré-mesurées stockées dans une ou plusieurs feuilles blisters.

5. Dispositif selon l'une quelconque des revendications précédentes, dans lequel la formulation de poudre sèche est produite par un processus de micronisation.

6. Dispositif selon l'une quelconque des revendications précédentes, dans lequel la formulation de poudre sèche a une densité tassée de plus de 0,1 g/cm³(g/cc), de préférence dans lequel la formulation a une densité tassée de plus de 0,2 g/cm³(g/cc), plus préférablement dans lequel la formulation a une densité tassée de plus de 0,5 g/cm³(g/cc).

7. Dispositif selon l'une quelconque des revendications précédentes, dans lequel l'agent pharmaceutiquement actif a un effet systémique après l'administration par inhalation pulmonaire.

8. Dispositif selon l'une quelconque des revendications précédentes, dans lequel l'agent pharmaceutiquement actif est sélectionné parmi l'héparine, l'apomorphine, le Glycopyrrolate, la clomipramine ou le clobozam.

9. Dispositif selon l'une quelconque des revendications précédentes, dans lequel la formulation de poudre sèche est traitée sans l'utilisation d'un solvant organique.

10. Dispositif selon l'une quelconque des revendications précédentes, dans lequel la formulation de poudre sèche est traitée à sec en l'absence de tout solvant.

11. Dispositif selon l'une quelconque des revendications 1 à 8, dans lequel le véhicule est du lactose.

12. Dispositif selon l'une quelconque des revendications 1 à 11, dans lequel le véhicule est du lactose et l'additif est du stéarate de magnésium.

13. Dispositif tel que décrit dans l'une quelconque des revendications 1 à 12 dans lequel une pluralité d'agents actifs sont présents dans la formulation.

14. Particule active composite pour une utilisation dans une formulation de poudre sèche pour inhalation pulmonaire, dans laquelle la formulation est dans un inhalateur, et dans laquelle la particule active composite est préparée en utilisant un procédé comprenant un broyage par jet des particules actives en présence de particules de matériau additif, dans laquelle le matériau additif est un stéarate métallique, et dans laquelle la formulation comprend des particules véhicules qui ont également un matériau additif appliqué à leurs surfaces éventuellement par simple mélange léger ou co-broyage
